(19) Europäisches Patentamt
European Patent Office
Office européen des brevets

(11) EP 2 952 195 A1

(12) EUROPEAN PATENT APPLICATION

(43) Date of publication:
**09.12.2015 Bulletin 2015/50**

(51) Int Cl.:
***A61K 31/546*** *(2006.01)* ***A61P 31/04*** *(2006.01)*

(21) Application number: **15166135.2**

(22) Date of filing: **19.09.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **21.09.2007 US 974194 P**

(62) Document number(s) of the earlier application(s) in accordance with Art. 76 EPC:
**08832124.5 / 2 200 618**

(71) Applicant: **AstraZeneca AB**
**151 85 Södertälje (SE)**

(72) Inventors:
- **Dedhiya, Mehendra**
**Pomona, NY New York 10970 (US)**
- **Ge, Yigong**
**Belmont, CA California 94002 (US)**

(74) Representative: **Burns, Tracy Anne**
**AstraZeneca**
**Intellectual Property**
**Alderley Park**
**Macclesfield, Cheshire SK10 4TG (GB)**

Remarks:
This application was filed on 01-05-2015 as a divisional application to the application mentioned under INID code 62.

# (54) SOLUBLE DOSAGE FORMS CONTAINING CEPHEM DERIVATIVES SUITABLE FOR PARENTERAL ADMINISTRATION

(57) The present invention relates to new dosage forms of cephem compounds, useful for the treatment of bacterial infections. The dosage forms are stable, exhibit enhanced solubility, and are particularly well suited for, e.g., parenteral administration.

EP 2 952 195 A1

## Description

This application claims the benefit of U.S. Provisional Application No. 60/974,194, filed September 21, 2007, the entire contents of which are hereby incorporated by reference.

## FIELD OF THE INVENTION

The present invention relates to new dosage forms of cephem compounds, useful for the treatment of bacterial infections. The dosage forms are stable, exhibit enhanced solubility, and are particularly well suited for, e.g., parenteral administration.

## BACKGROUND OF THE INVENTION

U.S. Patent No. 6,417,175 discloses phosphonocephem derivatives having excellent antibacterial activities for a broad range of Gram-positive and Gram-negative bacteria. These compounds are of the general formula:

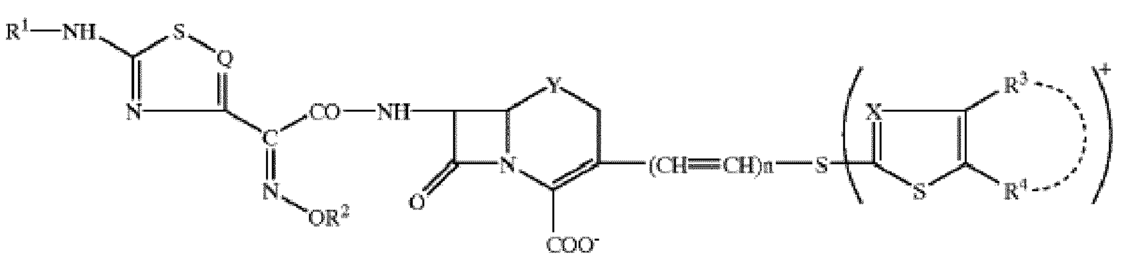

wherein $R^1$-$R^4$, Q, X, Y and n are as defined therein. One such compound is 7β-[2(Z)-ethoxyimino-2-(5-phosphonoamino-1,2,4-thiadiazol-3-yl)acetamido]-3-[4-(1-methyl-4-pyridinio)-2-thiazolythio]-3-cephem-4-carboxylate. U.S. Patent No. 6,417,175 discloses methods for preparing this compound (see, e.g., Examples 1, 2, 5 and 6), and generically discloses formulations of the compounds described therein.

U.S. Patent No. 6,906,055 discloses compounds of formula:

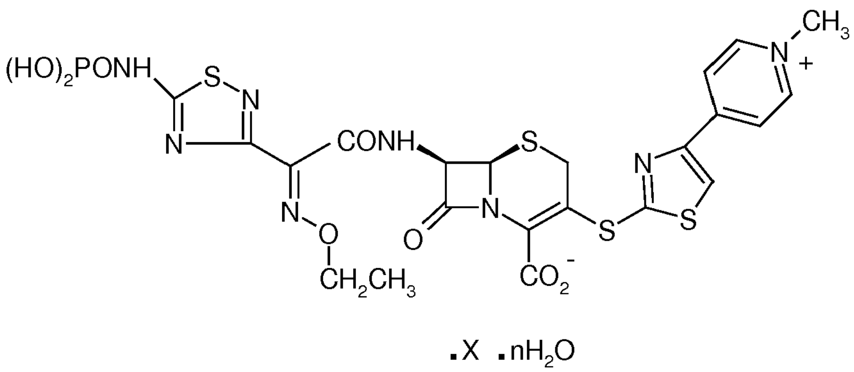

in which X is $CH_3COOH$, $CH_3CH_2COOH$ or $CH_3CN$ and n is 0-5. One such compound (where X is $CH_3COOH$ and n is 1) is (6R,7R)-7-[[2(Z)-ethoxyimino-[5-(phosphonoamino)-1,2,4-thiadiazol-3-yl]acetyl]amino]-3-[[4-(1-methyl-pyridinium-4-yl)thiazol-2-yl]sulfanyl]-8-oxo-5-thia-1-azabicyclo[4.2.0]oct-2-en-2-carboxylate monoacetate monohydrate, which is also known as pyridinium, 4-[2-[[(6R,7R)-2-carboxy-7-[[2(Z)-ethoxyimino-[5-(phosphonoamino)-1,2,4-thiadiazol-3-yl]acetyl]amino]-8-oxo-5-thia-1-azabicyclo[4.2.0]oct-2-en-3-yl]thio-4-thiazolyl]-1-methyl-, inner salt, monoacetate, monohydrate (molecular formula $C_{22}H_{21}N_8O_8PS_4$ . $C_2H_4O_2$ . $H_2O$, molecular weight 762.75). The USAN name for this compound is ceftaroline fosamil.

The INN (WHO Drug Information, Vol. 21, No2, 2007) proposed name ceftaroline fosamil refers to the following compound: 4-[2-[[(6R,7R)-2-carboxy-7-[[2(Z)-ethoxyimino-[5-(phosphonoamino)-1,2,4-thiadiazol-3-yl]acetyl]amino]-8-oxo-5-thia-1-azabicyclo[4.2.0]oct-2-en-3-yl]thio-4-thiazolyl]-1-methyl-, inner salt. The INN name ceftaroline fosamil refers to ceftaroline fosamil on an anhydrous, acetate free corrected basis (molecular formula $C_{22}H_{21}N_8O_8PS_4$, molecular weight 684.68)

When administered parenterally (such as by intravenous, intramuscular or subcutaneous administration), prodrugs, such as 7β-[2(Z)-ethoxyimino-2-(5-phosphonoamino-1,2,4-thiadiazol-3-yl)acetamido]-3-[4-(1-methyl-4-pyridinio)-2-thiazolythio]-3-cephem-4-carboxylate and ceftaroline fosamil (USAN and INN), are converted by body fluids into the active antibacterial moiety ceftaroline (molecular formula $C_{22}H_{22}N_8O_5S_4$, molecular weight 604.71)

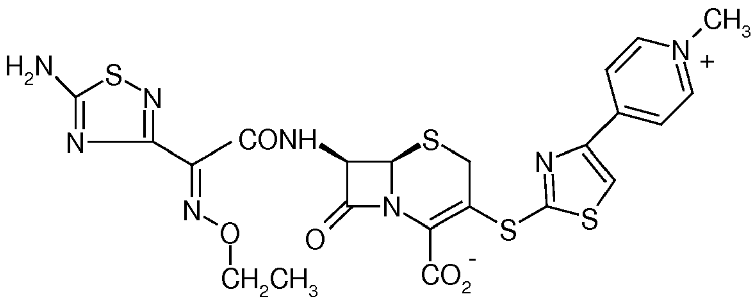

Antibacterial compounds may be administered by several routes, including parenterally, for example, by intravenous (IV) bolus, IV infusion and by intramuscular (IM) injection. Adsorption of the drug is dependent on its bioavailability. Drugs administered intravenously directly enter the systemic circulation, and are typically assumed to be 100% bioavailable. However, drugs administered intramuscularly must cross one or more biological membranes to reach the systemic circulation. It is desirable to have the same bioavailabilty (i.e, the same are under the curve (AUC)) for all parenteral dosage forms. However, the pharmacokinetic profiles for IV and IM formulations may be different, and obtaining desirable bioavailability (i.e., AUC) following intramuscular administration is not straightforward. For example, perfusion (blood flow per gram of tissue) greatly affects capillary absorption of small molecules when administered intramuscularly. Thus, the absorption site can affect the absorption rate. Also, absorption of the drug after IM administration may be delayed or erratic for salts of poorly soluble bases and acids. In addition, an IM formulation or dosage form must have sufficient solubility to be able to deliver the required dose in a small injectable volume with minimal local irritation. These limitations must be successfully overcome during the development of formulations for IM administration. IM administration is necessary, in some cases, e.g., in emergency rooms and in nursing facilities, where infusion is not advisable.

Further, for drugs with a short half life, bolus IV administration typically leads to fast elimination of the drug from a patient's system. Slow IV infusion of a dosage form may be desirable in such cases. However, the dosage form must be stable in and compatible with the IV fluid (e.g., 0.9% sodium chloride solution or 5% sugar solution) for the duration of the treatment. Thus, there is also a need to develop dosage forms having enhanced solubility for use in IV administration where administration of large volumes of infusion solution may not be preferred e.g., IV administration to infants, children and the elderly.

Accordingly, there remains a need in the art to provide new dosage forms containing cephem compounds which are stable, bioavailable and exhibit suitable pharmacokinetic parameters when administered, e.g., parenterally.

Applicants have developed dosage forms containing cephem compounds, such as ceftaroline fosamil, having enhanced solubility that are suitable for parenteral e.g., IV and IM administrations. The dosage forms are stable and exhibit excellent pharmacokinetic parameters when administered, for example, intramuscularly or intravenously.

## SUMMARY OF THE INVENTION

The present invention relates to new dosage forms of cephem compounds, wherein the active agent has enhanced solubility. The dosage forms are particularly well suited for parenteral (e.g., intravenous and intramuscular) administration.

In one embodiment, dosage forms comprising ceftaroline, or a pharmaceutically acceptable salt and/or solvate and/or prodrug thereof, and a solubilizing agent, wherein the molarity of the solubilizing agent in an aqueous solution of the dosage form is greater than about 0.1 M are described.

In further embodiments, dosage forms comprising ceftaroline, or a pharmaceutically acceptable salt and/or solvate and/or prodrug thereof, in which the active agent has a solubility greater than about 40 mg/mL are described.

In certain embodiments, the dosage form comprises a prodrug of ceftaroline, e.g., ceftaroline fosamil.

In additional embodiments, dosage forms comprising about 223 to about 2005 mg of ceftaroline fosamil, wherein a single dose parenteral administration of the dosage form provides an *in vivo* plasma profile for ceftaroline comprising a mean a mean $AUC_{0-\infty}$ of more than about 10650 ng.hr/mL.

In additional embodiments, dosage forms comprising about 223 to about 2005 mg of ceftaroline fosamil, wherein a single dose IM administration of the dosage form provides an *in vivo* plasma profile for ceftaroline comprising a mean a mean $AUC_{0-\infty}$ of more than about 10650 ng.hr/mL and a mean $C_{max}$ of less than about 39500 ng/mL are described.

In additional embodiments, dosage forms comprising about 223 to about 2005 mg of ceftaroline fosamil, wherein a single dose IM administration of the dosage form provides an *in vivo* plasma profile for ceftaroline comprising a mean

a mean $AUC_{0-\infty}$ of more than about 10650 ng.hr/mL, a mean $C_{max}$ of less than about 39500 ng/mL and a mean $T_{max}$ of about 1 or more hours are described.

Methods of treatment using the soluble dosage forms are also described.

## DETAILED DESCRIPTION OF THE INVENTION

The present invention relates to new dosage forms of cephem compounds which are stable, exhibit enhanced solubility, and are particularly well suited for, e.g., parenteral (e.g., IV, IM) administration.

Ceftaroline is an active antibacterial compound useful for treating a broad range of Gram-positive and Gram-negative bacteria. However, the aqueous solubility of ceftaroline is limited (~ 2-3 mg/mL) and is, therefore, too low to enable ceftaroline to be used directly in parenteral formulations. For example, the maximum dosage of ceftaroline that could be administered from a 100 mL volume IV infusion bag is only about 200-300 mg.

Ceftaroline fosamil, a prodrug of ceftaroline, has a higher aqueous solubility (about 36 mg/mL). Although the solubility of the prodrug is greater than that for the active moiety ceftaroline, the aqueous solubility of ceftaroline fosamil is still not sufficient to enable ceftaroline fosamil to be used directly for IM administration, where volumes administered are typically 5 mL or less per site. For example, the maximum dosage of ceftaroline fosamil that could be administered intramuscularly using 5 mL of solution is only about 180 mg per site.

Applicants have discovered that highly soluble dosage forms containing cephem compounds, such as ceftaroline fosamil, may be prepared. The soluble dosage forms are therefore useful for parenteral (both IV and IM) administration and enable higher doses of the active ingredient to be administered using smaller solution volumes. The dosage forms comprise a cephem compound, e.g., ceftaroline or a pharmaceutically acceptable salt and/or solvate and/or prodrug thereof, as active agent and a solubilizing agent, wherein the solubilizing agent is present at a molarity such that the solubility of the active agent is increased. For example, the solubility of the active agent is increased relative to a corresponding dosage form that does not contain the solubilizing agent.

In one aspect, the present invention relates to dosage forms comprising ceftaroline, or a pharmaceutically acceptable salt and/or solvate and/or prodrug thereof, (e.g., ceftaroline fosamil) and a solubilizing agent wherein the solubilizing agent is present at a molarity greater than about 0.1 M.

In additional embodiments, the solubilizing agent is present at a molarity greater than about 0.2 M, greater than about 0.3 M, greater than about 0.4 M, greater than about 0.5 M, greater than about 0.6 M, greater than about 0.7 M, greater than about 0.8 M, greater than about 0.9 M, greater than about 1.0 M, greater than about 1.1 M, greater than about 1.2 M, greater than about 1.3 M, greater than about 1.4 M, greater than about 1.5 M, greater than about 1.75 M, greater than about 2.0 M, greater than about 2.3 M or greater than about 2.5 M.

In additional embodiments, the solubilizing agent is present at a molarity of about 0.5 M, about 0.6 M, about 0.7 M, about 0.8 M, about 0.9 M about 1.0 M, about 1.1 M, about 1.2 M, about 1.3 M, about 1.4 M, about 1.5 M, about 1.6 M, about 1.7 M, about 1.8 M, about 1.9 M, about 2.0 M about 2.3 M or about 2.5 M. For example, the solubilizing agent is present at a molarity of about 0.5 M, about 1.0 M, about 1.5 M, about 2.0 M or about 2.3 M.

Suitable solubilizing agents include, but are not limited to, acids, such as carboxylic acids, amino acids. For example, the solubilizing may be selected from saturated carboxylic acids, unsaturated carboxylic acids, fatty acids, keto acids, aromatic carboxylic acids, dicarboxylic acids, tricarboxylic acids, $\alpha$-hydroxy acids, amino acids, and combinations thereof.

Specific solubilizing agents that may be used include, but are not limited to, formic acid, acetic acid, propionic acid, butyric acid, valeric acid, caproic acid, enanthic acid, caprylic acid, pelargonic acid, capric acid, lauric acid, stearic acid, acrylic acid, docosahexaenoci acid, eicosapentaenoic acid, pyruvic acid, benzoic acid, salicylic acid, aldaric acid, oxalic acid, malonic acid, malic acid, succinic acid, glutaric acid, adipic acid, citric acid, lactic acid, alanine, arginine, aspargine, aspartic acid, cysteine, glutamine, glycine, histidine, isoleucine, leucine, lysine, methionine, phenylalanine, praline, serine, threonine, tryptophan, tyrosine, valine, and combinations thereof (including salts thereof and/or individual stereoisomers and/or mixtures of stereoisomers thereof).

In certain embodiments, the solubilizing agent is selected from acetic acid, salts thereof and combinations thereof, (e.g., acetic acid/sodium acetate), citric acid, salts thereof and combinations thereof (e.g., citric acid/sodium citrate), DL arginine, L-arginine and histadine. In one embodiment, the solubilizing agent is DL-arginine. In one embodiment, the solubilizing agent is L-arginine. In one embodiment, the solubilizing agent is acetic acid/sodium acetate. In one embodiment, the solubilizing agent is citric acid/sodium citrate.

In additional embodiments, the solubility of the active agent in the dosage form is greater than about 40 mg/mL, such as greater than about 50 mg/mL, greater than about 75 mg/mL, greater than about 100 mg/mL, greater than about 125 mg/mL, greater than about 150 mg/mL, greater than about 175 mg/mL, greater than about 200 mg/mL or greater than about 250 mg/mL, when measured, for example, in water at 25 °C.

In additional embodiments, the solubility of the active agent in the dosage form is from about 100 to about 250 mg/mL, from about 150 to about 250 mg/ml, from about 180 to about 200 mg/mL or from about 200 to about 250 mg/mL,

when measured, for example, in water at 25 °C.

In certain embodiments, the dosage form comprises a prodrug of ceftaroline, e.g., ceftaroline fosamil. Examples of suitable dosage forms are given in Tables 1-4.

**Table 1: Dosage Forms Containing L-Arginine**

| Ingredient | Range (mg) | Preferred Range (mg) | Example 1* (mg) | Example 2** (mg) | Example 3*** (mg) |
|---|---|---|---|---|---|
| Ceftaroline fosamil[a] | 100-2200 | 200- 1400 | 668 | 668 | 668 |
| L-Arginine | 26-1350 | 50 - 800 | 400 | 348 | 174 |

* 2.3 M L-arginine; ** 2.0 M L-arginine; *** 1.0 M L-arginine
[a] A dose of about 668 mg of ceftaroline fosamil (USAN) is equivalent to a dose of about 530 mg of ceftaroline

**Table 2: Dosage Forms Containing DL-Arginine**

| Ingredient | Range (mg) | Preferred Range (mg) | Example 1* (mg) | Example 2** (mg) | Example 3*** (mg) |
|---|---|---|---|---|---|
| Ceftaroline fosamil[a] | 100-2200 | 200 - 1400 | 668 | 668 | 668 |
| DL-Arginine | 26-1350 | 50- 800 | 400 | 348 | 174 |

* 2.3 M DL-arginine; ** 2.0 M DL-arginine; *** 1.0 M DL-arginine
[a] A dose of about 668 mg of ceftaroline fosamil (USAN) is equivalent to a dose of about 530 mg of ceftaroline

**Table 3: Dosage Forms Containing Acetic Acid/Sodium Acetate**

| Ingredient | Range (mg) | Preferred Range (mg) | Example 1* (mg) | Example 2** (mg) |
|---|---|---|---|---|
| Ceftaroline fosamil[a] | 100-2200 | 200-1400 | 668 | 668 |
| Acetic Acid/Sodium Acetate | 10-550 | 24-300 | 164 | 82 |

* 2.0 M acetic acid/acetate; ** 1.0 M acetic acid/acetate
[a] A dose of about 668 mg of ceftaroline fosamil (USAN) is equivalent to a dose of about 530 mg of ceftaroline

**Table 4: Dosage Forms Containing Citric Acid/Sodium Citrate**

| Ingredient | Range (mg) | Preferred Range (mg) | Example 1* (mg) | Example 2** (mg) |
|---|---|---|---|---|
| Ceftaroline fosamil[a] | 100-2200 | 200-1400 | 668 | 668 |
| Citric Acid/Sodium Citrate | 40-550 | 80-1200 | 588 | 294 |

* 2.0 M citric acid/citrate; ** 1.0 M citric acid/citrate
[a] A dose of about 668 mg of ceftaroline fosamil (USAN) is equivalent to a dose of about530 mg of ceftaroline

The dosage forms may be prepared, for example, by mixing a prodrug of the active agent (e.g., ceftaroline fosamil) and the solubilizing agent (e.g., DL arginine, L-arginine, citric acid/sodium citrate, acetic acid/sodium acetate) in a blender under sterile conditions until a uniform blend is obtained. Pre-sterilized vials may then be filled with an appropriate amount of the sterile blend. The predetermined amount of sterile blend may then be mixed with a solvent, e.g., water, saline, about 5-10 % sugar (e.g., glucose, dextrose) solution and combinations thereof prior to administration. In addition, the solution may be frozen and thawed prior to further processing.

The solubilizing agent may be used in solid or in solution form. When used in solid form, the solubilizing agent and the prodrug of the active ingredient (e.g., ceftaroline fosamil) may be mixed together as described above, then solvent added prior to parenteral administration. When used in solution form, the prodrug of the active ingredient (e.g., ceftaroline fosamil) may be mixed with a solution of the solubilizing agent prior to parenteral administration.

In further embodiments, the dosage form comprises from about 177 to about 2005 mg ceftaroline, or a pharmaceutically acceptable salt and/or solvate and/or prodrug thereof, such as from about 177 mg to about 1337 mg ceftaroline, or a pharmaceutically acceptable salt and/or solvate and/or prodrug thereof, for example from about 353 to about 891 mg ceftaroline, or a pharmaceutically acceptable salt and/or solvate and/or prodrug thereof, for further example from about 353 mg to about 668 mg of ceftaroline, or a pharmaceutically acceptable salt and/or solvate and/or prodrug thereof.

In further embodiments, the dosage form comprises from about 223 to about 2005 mg ceftaroline fosamil, such as from about 223 mg to about 1337 mg ceftaroline fosamil, for example from about 446 to about 891 mg ceftaroline fosamil, for further example from about 446 mg to about 668 mg of ceftaroline fosamil. For example, ceftaroline fosamil (USAN) (molecular formula $C_{22}H_{21}N_8O_8PS_4 . C_2H_4O_2 . H_2O$, molecular weight 762.75).

In one embodiment, the dosage form contains about 223 mg ceftaroline fosamil. In one embodiment, the dosage form contains about 446 mg ceftaroline fosamil. In one embodiment, the dosage form contains about 557 mg ceftaroline fosamil. In one embodiment, the dosage form contains about 668 mg ceftaroline fosamil. In one embodiment, the dosage form contains about 891 mg ceftaroline fosamil. In one embodiment, the dosage form contains about 1114 mg ceftaroline fosamil. In one embodiment, the dosage form contains about 1337 mg ceftaroline fosamil. In one embodiment, the dosage form contains about 2005 mg ceftaroline fosamil. For example, ceftaroline fosamil (USAN) (molecular formula $C_{22}H_{21}N_8O_8PS_4 . C_2H_4O_2 . H_2O$, molecular weight 762.75).

In one embodiment, the dosage form contains about 200 mg ceftaroline fosamil. In one embodiment, the dosage form contains about 400 mg ceftaroline fosamil. In one embodiment, the dosage form contains about 500 mg ceftaroline fosamil. In one embodiment, the dosage form contains about 600 mg ceftaroline fosamil. In one embodiment, the dosage form contains about 800 mg ceftaroline fosamil. In one embodiment, the dosage form contains about 1000 mg ceftaroline fosamil. In one embodiment, the dosage form contains about 1200 mg ceftaroline fosamil. In one embodiment, the dosage form contains about 1800 mg ceftaroline fosamil. For example, ceftaroline fosamil (INN) ( anhydrous, acetate free corrected basis, molecular formula $C_{22}H_{21}N_8O_8PS_4$, molecular weight 684.68)

In further embodiments, the dosage form comprises from about 177 to about 1589 mg ceftaroline, such as from about 177 mg to about 1060 mg ceftaroline, for example from about 353 to about 706 mg ceftaroline, for further example from about 353 mg to about 618 mg of ceftaroline, for further example from about 353 mg to about 530 mg of ceftaroline. In additional embodiments, the dosage form contains about 177 mg ceftaroline, about 353 mg ceftaroline, about 442 mg ceftaroline, about 530 mg ceftaroline, about 618 mg ceftaroline, about 706 mg ceftaroline, about 883 mg ceftaroline, about 1060 mg ceftaroline or about 1589 mg ceftaroline. For example ceftaroline, molecular formula $C_{22}H_{22}N_8O_5S_4$, molecular weight 604.71.

In one embodiment, the dosage form contains about 668 mg ceftaroline fosamil (USAN). In one embodiment, the dosage form contains about 600 mg ceftaroline fosamil (INN).

In one embodiment, the dosage form contains about 446 mg ceftaroline fosamil (USAN). In one embodiment, the dosage form contains about 400 mg ceftaroline fosamil (INN).

In one embodiment, the dosage form contains about 530 mg ceftaroline

In further embodiments, the dosage form contains about 353 mg ceftaroline.

In the clinical study of anti-infective drugs, dose selection, dose regimen, and duration of therapy should take into account the biopharmaceutics, pharmacokinetics, and pharmacodynamics of the anti-infective drug/drug product. See e.g., "Developing Antimicrobial Drugs - General Considerations for Clinical Trials," U.S. Department of Health and Human Services, Food and Drug Administration, Draft Guidance for Industry, July 1998.

Pharmacodynamics can establish the relationship between the dose of an anti-infective drug and its antimicrobial activity. A combined pharmacokinetic/pharmacodynamic (PK/PD) evaluation includes relating drug concentrations in plasma to the *in-vitro* susceptibility of the target microorganisms and/or clinical outcomes. Usually, plasma drug concentrations are related to the minimum inhibitory concentration (MIC). In addition, the drug concentration-time profile can be transformed to a single measure of exposure (e.g., area under the curve (AUC) or time above the minimum inhibitory concentration (T>MIC) and related to microbiological and/or clinical outcome to determine the optimal dosage regimen. The choice of pharmacodynamic variable (e.g., AUC/MIC, Peak Plasma Concentration ($C_{max}$)/MIC, T>MIC) depends upon the mechanism of antimicrobial effect.

The AUC is the measure of total exposure of the antibiotic drug to the circulation over time. The serum antibiotic concentration and the period of time the antibiotic concentration is above the MIC and considered to be pharmacokinetic properties of antimicrobials. The product of these two factors is represented by the area under the serum concentration-time curve (AUC). Bacterial killing is therefore a function of AUC.

For the development of antimicrobial drug dosage forms suitable for parenteral (e.g., IM) administration, the AUC values observed after IM administration of the dosage form should be similar to the AUC values observed for the drug when the dosage form is administered intravenously. Moreover, suitable MIC criteria must be met in order for IM administration of the drug to be effective.

When administered parenterally, the dosage forms described herein provide the following pharmacokinetic

parameters.

When administered intramuscularly, a time of maximum plasma concentration ($T_{max}$) for ceftaroline (active moiety) in human patients of about 1 or more hours (e.g., about 1.5 or more hours) is observed. In additional embodiments, a $T_{max}$ of ceftaroline (active moiety) in human patients ranging from between about 1 to about 4 hours, for example, between about 1 to about 3 hours, such between about 1.5 and about 2 hours is observed. In other embodiments, a $T_{max}$ for ceftaroline fosamil (prodrug) in human patients of about 0.05 or more hours is observed. The time of maximum plasma concentration is measured once infusion is complete.

(a) In one aspect, the present invention relates to a dosage form comprising from about 223 mg to about 2005 mg ceftaroline fosamil (USAN), wherein a single dose parenteral administration of the dosage form provides an *in vivo* plasma profile for ceftaroline comprising a mean $AUC_{0-\infty}$ of more than about 10650 ng.hr/mL.

(b) In one embodiment, the dosage form comprises from about 223 mg to about 2005 mg ceftaroline fosamil (USAN), wherein a single dose IM administration of the dosage form provides an *in vivo* plasma profile for ceftaroline comprising a mean $C_{max}$ of less than about 39500 ng/mL

(c) In another embodiment, the dosage form comprises from about 223 mg to about 2005 mg ceftaroline fosamil (USAN), wherein a single dose IM administration of the dosage form provides an *in vivo* plasma profile for ceftaroline comprising a a mean $AUC_{0-\infty}$ of more than about 10650 ng.hr/mL and a mean $C_{max}$ of less than about 39500 ng/mL.

(d) In a further embodiment, the dosage form comprises from about 223 mg to about 2005mg ceftaroline fosamil (USAN), wherein a single dose IM administration of the dosage form provides an *in vivo* plasma profile for ceftaroline comprising a mean $AUC_{0-\infty}$ of more than about 10650 ng.hr/mL, a mean $C_{max}$ of less than about 39500 ng/mL and a mean $T_{max}$ of about 1 or more hours.

(e) In another aspect, the present invention relates to a dosage form comprising about 223 mg ceftaroline fosamil (USAN), wherein a single dose parentral administration of the dosage form provides an *in vivo* plasma profile for ceftaroline comprising a mean $AUC_{0-\infty}$ of more than about 10650 ng.hr/mL.

(f) In one embodiment, the dosage form comprises about 223 mg ceftaroline fosamil (USAN), wherein a single dose IM administration of the dosage form provides an *in vivo* plasma profile for ceftaroline comprising a mean $C_{max}$ of less than about 4900 ng/mL.

(g) In another embodiment, the dosage form comprises about 223 mg ceftaroline fosamil (USAN), wherein a single dose IM administration of the dosage form provides an *in vivo* plasma profile for ceftaroline comprising a mean $AUC_{0-\infty}$ of more than about 10650 ng.hr/mL and a mean $C_{max}$ of less than about 4900 ng/mL.

(h) In a further embodiment, the dosage form comprises about 223 mg ceftaroline fosamil (USAN), wherein a single dose IM administration of the dosage form provides an *in vivo* plasma profile for ceftaroline comprising a mean $AUC_{0-\infty}$ of more than about 10650 ng.hr/mL, a mean $C_{max}$ of less than about 4900 ng/mL and a mean $T_{max}$ of about 1 or more hours.

(i) In another aspect, the present invention relates to a dosage form comprising about 446 mg ceftaroline fosamil (USAN), wherein a single dose parenteral administration of the dosage form provides an *in vivo* plasma profile for ceftaroline comprising a mean $AUC_{0-\infty}$ of more than about 21350 ng.hr/mL.

(j) In one embodiment, the dosage form comprises about 446 mg ceftaroline fosamil (USAN), wherein a single dose IM administration of the dosage form provides an *in vivo* plasma profile for ceftaroline comprising a mean $C_{max}$ of less than about 9800 ng/mL.

(k) In another embodiment, the dosage form comprises about 446 mg ceftaroline fosamil (USAN), wherein a single dose IM administration of the dosage form provides an *in vivo* plasma profile for ceftaroline comprising a mean $AUC_{0-\infty}$ of more than about 21350 ng.hr/mL and a mean $C_{max}$ of less than about 9800 ng/mL.

(l) In a further embodiment, the dosage form comprises about 446 mg ceftaroline fosamil (USAN), wherein a single dose IM administration of the dosage form provides an *in vivo* plasma profile for ceftaroline comprising a mean $AUC_{0-\infty}$ of more than about 21350 ng.hr/mL, a mean $C_{max}$ of less than about 9800 ng/mL and a mean $T_{max}$ of about 1 or more hours.

(m) In another aspect, the present invention relates to a dosage form comprising about 557 mg ceftaroline fosamil (USAN), wherein a single dose parenteral administration of the dosage form provides an *in vivo* plasma profile for ceftaroline comprising a mean $AUC_{0-\infty}$ of more than about 25800 ng.hr/mL.

(n) In one embodiment, the dosage form comprises about 557 mg ceftaroline fosamil (USAN), wherein a single dose IM administration of the dosage form provides an *in vivo* plasma profile for ceftaroline comprising a mean $C_{max}$ of less than about 11100 ng/mL.

(o) In another embodiment, the dosage form comprises about 557 mg ceftaroline fosamil (USAN), wherein a single dose IM administration of the dosage form provides an *in vivo* plasma profile for ceftaroline comprising a mean $AUC_{0-\infty}$ of more than about 25800 ng.hr/mL and a mean $C_{max}$ of less than about 11100 ng/mL.

(p) In a further embodiment, the dosage form comprises about 557 mg ceftaroline fosamil (USAN), wherein a single dose IM administration of the dosage form provides an *in vivo* plasma profile for ceftaroline comprising a mean $AUC_{0-\infty}$ of more than about 25800 ng.hr/mL, a mean $C_{max}$ of less than about 11100 ng/mL and a mean $T_{max}$ of about 1 or more hours.

(q) In another aspect, the present invention relates to a dosage form comprising about 668 mg ceftaroline fosamil (USAN), wherein a single dose parenteral administration of the dosage form provides an *in vivo* plasma profile for ceftaroline comprising a mean $AUC_{0-\infty}$ of more than about 28800 ng.hr/mL.

(r) In one embodiment, the dosage form comprises about 668 mg ceftaroline fosamil (USAN), wherein a single dose IM administration of the dosage form provides an *in vivo* plasma profile for ceftaroline comprising a mean $C_{max}$ of less than about 12000 ng/mL.

(s) In another embodiment, the dosage form comprises about 668 mg ceftaroline fosamil (USAN), wherein a single dose IM administration of the dosage form provides an *in vivo* plasma profile for ceftaroline comprising a mean $AUC_{0-\infty}$ of more than about 28800 ng.hr/mL and a mean $C_{max}$ of less than about 12000 ng/mL.

(t) In a further embodiment, the dosage form comprises about 668 mg ceftaroline fosamil (USAN), wherein a single dose IM administration of the dosage form provides an *in vivo* plasma profile for ceftaroline comprising a mean $AUC_{0-\infty}$ of more than about 28800 ng.hr/mL, a mean $C_{max}$ of less than about 12000 ng/mL and a mean $T_{max}$ of about 1 or more hours.

(u) In another aspect, the present invention relates to a dosage form comprising about 891 mg ceftaroline fosamil (USAN), wherein a single dose parenteral administration of the dosage form provides an *in vivo* plasma profile for ceftaroline comprising a mean $AUC_{0-\infty}$ of more than about 49000 ng.hr/mL.

(v) In one embodiment, the dosage form comprises about 891 mg ceftaroline fosamil (USAN), wherein a single dose IM administration of the dosage form provides an *in vivo* plasma profile for ceftaroline comprising a mean $C_{max}$ of less than about 17750 ng/mL.

(w) In another embodiment, the dosage form comprises about 891 mg ceftaroline fosamil (USAN), wherein a single dose IM administration of the dosage form provides an *in vivo* plasma profile for ceftaroline comprising a mean $AUC_{0-\infty}$ of more than about 49000 ng.hr/mL and a mean $C_{max}$ of less than about 17750 ng/mL.

(x) In a further embodiment, the dosage form comprises about 891 mg ceftaroline fosamil (USAN), wherein a single dose IM administration of the dosage form provides an *in vivo* plasma profile for ceftaroline comprising a mean $AUC_{0-\infty}$ of more than about 49000 ng.hr/mL, a mean $C_{max}$ of less than about 17750 ng/mL and a mean $T_{max}$ of about 1 or more hours.

(y) In another aspect, the present invention relates to a dosage form comprising about 1114 mg ceftaroline fosamil (USAN), wherein a single dose parenteral administration of the dosage form provides an *in vivo* plasma profile for ceftaroline comprising a mean $AUC_{0-\infty}$ of more than about 66000 ng.hr/mL.

(z) In one embodiment, the dosage form comprises about 1114 mg ceftaroline fosamil (USAN), wherein a single dose IM administration of the dosage form provides an *in vivo* plasma profile for ceftaroline comprising a mean $C_{max}$ of less than about 22500 ng/mL.

(aa) In another embodiment, the dosage form comprises about 1114 mg ceftaroline fosamil (USAN), wherein a single dose IM administration of the dosage form provides an *in vivo* plasma profile for ceftaroline comprising a mean $AUC_{0-\infty}$ of more than about 66000 ng.hr/mL and a mean $C_{max}$ of less than about 22500 ng/mL.

(bb) In a further embodiment, the dosage form comprises about 1114 mg ceftaroline fosamil (USAN), wherein a single dose IM administration of the dosage form provides an *in vivo* plasma profile for ceftaroline comprising a mean $AUC_{0-\infty}$ of more than about 66000 ng.hr/mL, a mean $C_{max}$ of less than about 22500 ng/mL and a mean $T_{max}$ of about 1 or more hours.

(cc) In another aspect, the present invention relates to a dosage form comprising about 1337 mg ceftaroline fosamil (USAN), wherein a single dose parenteral administration of the dosage form provides an *in vivo* plasma profile for ceftaroline comprising a mean $AUC_{0-\infty}$ of more than about 79500 ng.hr/mL.

(dd) In one embodiment, the dosage form comprises about 1337 mg ceftaroline fosamil (USAN), wherein a single dose IM administration of the dosage form provides an *in vivo* plasma profile for ceftaroline comprising a mean $C_{max}$ of less than about 26500 ng/mL.

(ee) In another embodiment, the dosage form comprises about 1337 mg ceftaroline fosamil (USAN), wherein a single dose IM administration of the dosage form provides an *in vivo* plasma profile for ceftaroline comprising a mean $AUC_{0-\infty}$ of more than about 79500 ng.hr/mL and a mean $C_{max}$ of less than about 26500 ng/mL.

(ff) In a further embodiment, the dosage form comprises about 1337 mg ceftaroline fosamil (USAN), wherein a single dose IM administration of the dosage form provides an *in vivo* plasma profile for ceftaroline comprising a mean $AUC_{0-\infty}$ of more than about 79500 ng.hr/mL, a mean $C_{max}$ of less than about 26500 ng/mL and a mean $T_{max}$ of about 1 or more hours.

(gg) In another aspect, the present invention relates to a dosage form comprising about 2005 mg ceftaroline fosamil (USAN), wherein a single dose parenteral administration of the dosage form provides an *in vivo* plasma profile for ceftaroline comprising a mean $AUC_{0-\infty}$ of more than about 126000 ng.hr/mL.

(hh) In one embodiment, the dosage form comprises about 2005 mg ceftaroline fosamil (USAN), wherein a single dose IM administration of the dosage form provides an *in vivo* plasma profile for ceftaroline comprising a mean $C_{max}$ of less than about 39500 ng/mL.

(ii) In another embodiment, the dosage form comprises about 2005 mg ceftaroline fosamil (USAN), wherein a single dose IM administration of the dosage form provides an *in vivo* plasma profile for ceftaroline comprising a mean $AUC_{0-\infty}$ of more than about 126000 ng.hr/mL and a mean $C_{max}$ of less than about 39500 ng/mL.

(jj) In a further embodiment, the dosage form comprises about 2005 mg ceftaroline fosamil (USAN), wherein a single dose IM administration of the dosage form provides an *in vivo* plasma profile for ceftaroline comprising a mean $AUC_{0-\infty}$ of more than about 126000 ng.hr/mL, a mean $C_{max}$ of less than about 39500 ng/mL and a mean $T_{max}$ of about 1 or more hours.

In additional embodiments, the dosage form of any of the embodiments described above (e.g., embodiments aa-jj) may comprise the corresponding amount of ceftaroline fosamil (INN) or ceftaroline.

One will recognize that a dose of about 223 mg ceftaroline fosamil (USAN) is equivalent to a dose of about 200 mg ceftaroline fosamil (INN) which is equivalent to a dose of about 177 mg ceftaroline.

A dose of about 446 mg ceftaroline fosamil (USAN) is equivalent to a dose of about 400 mg ceftaroline fosamil (INN) which is equivalent to a dose of about 353 mg ceftaroline.

A dose of about 557 mg ceftaroline fosamil (USAN) is equivalent to a dose of about 500 mg ceftaroline fosamil (INN) which is equivalent to a dose of about 442 mg ceftaroline.

A dose of about 668 mg ceftaroline fosamil (USAN) is equivalent to a dose of about 600 mg ceftaroline fosamil (INN) which is equivalent to a dose of about 530 mg ceftaroline.

A dose of about 891 mg ceftaroline fosamil (USAN) is equivalent to a dose of about 800 mg ceftaroline fosamil (INN) which is equivalent to a dose of about 706 mg ceftaroline.

A dose of about 1114 mg ceftaroline fosamil (USAN) is equivalent to a dose of about 1000 mg ceftaroline fosamil (INN) which is equivalent to a dose of about 883 mg ceftaroline.

A dose of about 1337 mg ceftaroline fosamil (USAN) is equivalent to a dose of about 1200 mg ceftaroline

fosamil (INN) which is equivalent to a dose of about 1060 mg ceftaroline.

A dose of about 2005 mg ceftaroline fosamil (USAN) is equivalent to a dose of about 1800 mg ceftaroline fosamil (INN) which is equivalent to a dose of about 1589 mg ceftaroline.

In certain embodiments, the dosage forms provide these pharmacokinetic parameters when administered parenterally. In one embodiment, the dosage forms provide these pharmacokinetic parameters when administered intramuscularly. In another embodiment, the dosage forms provide these pharmacokinetic parameters when administered intravenously. For example, when administered intramuscularly at a concentration of about 228 mg ceftaroline fosamil (INN)/mL, In another example, when administered intramuscularly at a concentration of about 165 mg ceftaroline fosamil (INN)/mL. In another example, when administered intravenously at a concentration of about 1.2 to about 12 mg ceftaroline fosamil (INN)/mL.

In one embodiment, the dosage form comprises ceftaroline fosamil and L-arginine. In another embodiment, the dosage form consists essentially of ceftaroline fosamil and L-arginine. In a further embodiment, the dosage form consists of ceftaroline fosamil and L-arginine.

In one embodiment, the dosage form comprises ceftaroline fosamil and DL-arginine. In another embodiment, the dosage form consists essentially of ceftaroline fosamil and DL-arginine. In a further embodiment, the dosage form consists of ceftaroline fosamil and DL-arginine.

In one embodiment, the dosage form comprises ceftaroline fosamil and acetic acid/sodium acetate. In another embodiment, the dosage form consists essentially of ceftaroline fosamil and acetic acid/sodium acetate. In a further embodiment, the dosage form consists of ceftaroline fosamil and acetic acid/sodium acetate.

In one embodiment, the dosage form comprises ceftaroline fosamil and citric acid/sodium citrate. In another embodiment, the dosage form consists essentially of ceftaroline fosamil and citric acid/sodium citrate. In a further embodiment, the dosage form consists of ceftaroline fosamil and citric acid/sodium citrate.

In certain embodiments, the dosage form is a dry powder. In additional embodiments, the dosage form further comprises a solvent, such as water, physiological saline, about 5% to about 10 % glucose or dextrose solution, and combinations thereof.

In certain embodiments, the dosage form comprises about 668 mg ceftaroline fosamil and about 400 mg L-arginine, about 668 mg ceftaroline fosamil and about 348 mg L-arginine, about 668 mg ceftaroline fosamil and about 174 mg L-arginine.

In certain embodiments, the dosage form comprises about 668 mg ceftaroline fosamil and about 400 mg DL-arginine, about 668 mg ceftaroline fosamil and about 348 mg DL-arginine, about 668 mg ceftaroline fosamil and about 174 mg DL-arginine.

In certain embodiments, the dosage form comprises about 668 mg ceftaroline fosamil and about 164 mg acetic acid/sodium acetate, about 668 mg ceftaroline fosamil and about 120 mg acetic acid/sodium acetate, about 668 mg ceftaroline fosamil and about 82 mg acetic acid/sodium acetate.

In certain embodiments, the dosage form comprises about 668 mg ceftaroline fosamil and about 558 mg citric acid/sodium citrate, about 668 mg ceftaroline fosamil and about 440 mg citric acid/sodium citrate, about 668 mg ceftaroline fosamil and about 294 mg citric acid/sodium citrate.

In certain embodiments, the dosage form comprises about 446 mg ceftaroline fosamil and about 267 mg L-arginine, about 446 mg ceftaroline fosamil and about 230 mg L-arginine, about 446 mg ceftaroline fosamil and about 116 mg L-arginine.

In certain embodiments, the dosage form comprises about 446 mg ceftaroline fosamil and about 267 mg DL-arginine, about 446 mg ceftaroline fosamil and about 230 mg DL-arginine, about 446 mg ceftaroline fosamil and about 116 mg DL-arginine.

In certain embodiments, the dosage form comprises about 446 mg ceftaroline fosamil and about 110 mg acetic acid/sodium acetate, about 446 mg ceftaroline fosamil and about 82 mg acetic acid/sodium acetate, about 446 mg ceftaroline fosamil and about 55 mg acetic acid/sodium acetate.

In certain embodiments, the dosage form comprises about 446 mg ceftaroline fosamil and about 374 mg citric acid/sodium citrate, comprises about 446 mg ceftaroline fosamil and about 293 mg citric acid/sodium citrate, about 446 mg ceftaroline fosamil and about 197 mg citric acid/sodium citrate.

In certain embodiments, the drug (ceftaroline) or its prodrug (e.g. ceftaroline fosamil) and solubilizing agent are in the form of a solid (e.g., dry powder). In other embodiments, the drug or prodrug and solubilizing agent are in the form of a solution. In further embodiments, the drug (ceftaroline) or its prodrug (e.g. ceftaroline fosamil) and solubilizing agent are in the form of a slurry.

In certain embodiments, the solubilizing agent(s) is/are in liquid form. The drug (ceftaroline) or its prodrug (e.g., ceftaroline fosamil) may be mixed with the liquid solubilizing agent (either with or without an additional solvent being added) prior to parenteral administration.

Methods of Treatment

Ceftaroline fosamil (USAN, molecular formula of $C_{22}H_{21}N_8O_8PS_4 . C_2H_4O_2 . H_2O$) and ceftaroline fosamil (INN, anhydrous, acetate free, molecular formula $C_{22}H_{21}N_8O_8PS_4$, molecular weight 684.68) are N-phosphonoamino prodrugs of ceftaroline (molecular formula of $C_{22}H_{22}N_8O_5S_4$). Ceftaroline displays broad antibacterial potency against aerobic and some anaerobic Gram-positive and Gram-negative bacteria. In particular, ceftaroline has excellent activity against multiple drug-resistant staphylococci, including methicillin-resistant *Staphylococcus aureus* (MSRA), vancomycin-intermediate-susceptible *S. aureus* (VISA), vancomycin-resistant *S. aureus* (VSRA) and methicillin-resistant or vancomycin-intermediate-susceptible coagulase-negative *staphylococci* (MR-CoNS or VI-CoNS). Its in vitro antimicrobial spectrum also includes etiologic pathogens involved in respiratory and other nosocomial infections, such as *streptococci* (including penicillin-resistant *Streptococcus pneumoniae* [PRSP]), ampicillin-resistant *Haemophilius influenza, Monraxella catarrhalis,* the majority of pathogenic enteric bacilli, and selected anaerobic species. The minimum inhibitory concentration which inhibits 90% of the microbial strains in a given species (MIC90) is usually ≤ 2 mg/mL. Accordingly, the dosage forms of the present invention can be used to treat a wide range of bacterial infections in a patient, such as respiratory infections and urinary tract infections.

In a further aspect, the present invention relates to methods of treating bacterial infections by administering to a patient in need thereof a dosage form according to one or more of the embodiments recited above. In each case, in additional embodiments, the dosage form is administered parenterally (e.g., intravenously, intramuscularly) as a solution or suspension in a solvent, such as water, physiological saline, about a 5 % to about 10 % sugar (e.g., glucose, dextrose) solution, and combinations thereof.

Definitions

Unless defined otherwise, all technical and scientific terms used herein generally have the same meaning as commonly understood by one of ordinary skill in the art to which this invention belongs.

The term "about" or "approximately" as used herein means within an acceptable error range for the particular value as determined by one of ordinary skill in the art, which will depend in part on how the value is measured or determined, i.e., the limitations of the measurement system. For example, "about" can mean within 1 or more than 1 standard deviations, per the practice in the art. Alternatively, "about" can mean a range of up to 20%, and preferably up to 10% of a given value.

The term "bioavailability" refers to the extent to which the active ingredient or active moiety is absorbed from a drug product and becomes systematically available.

The term "effective amount" means the amount of the dosage form, which when administered to a patient (e.g., a mammal) for treating a disease, contains sufficient active ingredient to effect such treatment for the disease, so as to achieve the objectives of the invention. The "effective amount" will vary depending on the compound, the disease and its severity and the age, weight, physical condition and responsiveness, etc., of the patient to be treated.

The pharmacokinetic parameters described herein include area under the plasma concentration-time curve ($AUC_{0-t}$ and $AUC_{0-\infty}$), maximum plasma concentration ($C_{max}$), and time of maximum plasma concentration ($T_{max}$). Terminal elimination half-life ($T_{1/2}$) may also be provided. The time of maximum concentration, $T_{max}$. is determined as the time corresponding to $C_{max}$. Area under the plasma concentration-time curve up to the time corresponding to the last measurable concentration ($AUC_{0-t}$) is calculated by numerical integration using the linear trapezoidal rule as follows:

$$AUC_{0-t} = \sum_{i=2}^{n} 0.5 \cdot (C_i + C_{i-1}) \cdot (t_i - t_{i-1}) \qquad Eq.\ 1$$

where $C_i$ is the plasma memantine concentrations at the corresponding sampling time point $t_i$ and n is the number of time points up to and including the last quantifiable concentration.

The terminal half-life ($T_{1/2}$) is calculated using the following equation:

$$T_{1/2} = \frac{0.693}{\lambda_z} \qquad Eq.\ 2$$

where $\lambda_z$ is the terminal elimination rate constant.

The area under the plasma concentration-time curve from time zero to infinity is calculated according to the following equation:

$$AUC_{0-\infty} = AUC_{0-t} + \frac{C_{last}}{\lambda_z} \qquad Eq. 3$$

where $C_{last}$ is the last measurable concentration.

The terms "treat," "treatment," and "treating" refer to one or more of the following:

(a) relieving or alleviating at least one symptom of a disorder in a subject, including for example, allergic and inflammatory disorders, such as asthma and COPD;

(b) relieving or alleviating the intensity and/or duration of a manifestation of a disorder experienced by a subject including, but not limited to, those that are in response to a given stimulus (e.g., pressure, tissue injury, cold temperature, etc.);

(c) arresting, delaying the onset (i.e., the period prior to clinical manifestation of a disorder) and/or reducing the risk of developing or worsening a disorder.

A subject or patient in whom administration of the therapeutic compound is an effective therapeutic regimen for a disease or disorder is preferably a human, but can be any animal, including a laboratory animal in the context of a clinical trial or screening or activity experiment. Thus, as can be readily appreciated by one of ordinary skill in the art, the methods, compounds and compositions of the present invention are particularly suited to administration to any animal, particularly a mammal, and including, but by no means limited to, humans, domestic animals, such as feline or canine subjects, farm animals, such as but not limited to bovine, equine, caprine, ovine, and porcine subjects, wild animals (whether in the wild or in a zoological garden), research animals, such as mice, rats, rabbits, goats, sheep, pigs, dogs, cats, etc., avian species, such as chickens, turkeys, songbirds, etc., i.e., for veterinary medical use.

Pharmaceutically acceptable salts include those obtained by reacting the main compound, functioning as a base with an inorganic or organic acid to form a salt, for example, salts of hydrochloric acid, sulfuric acid, phosphoric acid, methane sulfonic acid, camphor sulfonic acid, oxalic acid, maleic acid, succinic acid, citric acid, formic acid, hydrobromic acid, benzoic acid, tartaric acid, fumaric acid, salicylic acid, mandelic acid, and carbonic acid. Pharmaceutically acceptable salts also include those in which the main compound functions as an acid and is reacted with an appropriate base to form, e.g., sodium, potassium, calcium, magnesium, ammonium, and choline salts. Those skilled in the art will further recognize that acid addition salts may be prepared by reaction of a compound with the appropriate inorganic or organic acid via any of a number of known methods. Alternatively, alkali and alkaline earth metal salts can be prepared by reacting a compound with the appropriate base via a variety of known methods. The following are further examples of acid salts that can be obtained by reaction with inorganic or organic acids: acetates, adipates, alginates, citrates, aspartates, benzoates, benzenesulfonates, bisulfates, butyrates, camphorates, digluconates, cyclopentanepropionates, dodecylsulfates, ethanesulfonates, glucoheptanoates, glycerophosphates, hemisulfates, heptanoates, hexanoates, fumarates, hydrobromides, hydroiodides, 2-hydroxy-ethanesulfonates, lactates, maleates, methanesulfonates, nicotinates, 2-naphthalenesulfonates, oxalates, palmoates, pectinates, persulfates, 3-phenylpropionates, picrates, pivalates, propionates, succinates, tartrates, thiocyanates, tosylates, mesylates and undecanoates.

The term "prodrug" means a compound that is a drug precursor which upon administration to a subject undergoes chemical conversion by metabolic or chemical processes to yield a compound an active moiety. Suitable prodrugs of ceftaroline include, e.g., ceftaroline fosamil (USAN, INN) and 7β-[2(Z)-ethoxyimino-2-(5-phosphonoamino-1,2,4-thiadiazol-3-yl)acetamido]-3-[4-(1-methyl-4-pyridinio)-2-thiazolythio]-3-cephem-4-carboxylate.

Solvates of a compound may form when a solvent molecule(s) is incorporated into the crystalline lattice structure of the compound molecule during, for example, a crystallization process. Suitable solvates include, e.g., hydrates (monohydrate, sesquihydrate, dihydrate), solvates with organic compounds (e.g., $CH_3CO_2H$, $CH_3CH_2CO_2H$, $CH_3CN$), and combinations thereof.

**EXAMPLES**

The present invention will now be further described by way of the following nonlimiting examples. In applying the disclosure of these examples, it should be kept clearly in mind that the examples are merely illustrative of the present invention and should not be construed as limiting the scope of the invention in any way as many variations and equivalents that are encompassed by the present invention will become apparent to those skilled in the art upon reading the present

disclosure.

Ceftaroline fosamil may be prepared as described in U.S. Patent No. 6,906,055.

**Example 1: pH Solubility Profile of Ceftaroline Fosamil**

The one hour and three hour kinetic solubility of ceftaroline fosamil was measured at room temperature (25 °C) by adding an excess of ceftaroline fosamil to different USP buffers with pH ranging from 1.2 to 9.0 at 0.05 M. The results are shown in Table 5.

**TABLE 5: pH Solubility Profile of Ceftaroline Fosamil (0.05 M)**

| pH | Solubility at 1 hr (mg/mL) | Solubility at 3 hrs (mg/mL) |
|---|---|---|
| 1.2 | 6 | 6 |
| 1.9 | 9 | 9 |
| 2.9 | 27 | 27 |
| 3.7 | 36 | 29 |
| 5.3 | 26 | 26 |
| 5.9 | 34 | 29 |
| 7.3 | 25 | 24 |
| 8.0 | 29 | 24 |
| 9.0 | 16 | 12 |
| USP Buffer Ranges: pH =1-2: HCl/KCl; pH = 3-5: sodium acetate; pH = 6-9: sodium phosphate | | |

As can be seen from Table 5, the solubility of ceftaroline fosamil ranges from about 25 mg/mL to about 36 mg/mL over a wide range of pH (about 3 to about 8, 0.05 M).

**Example 2: Solubility of Ceftaroline Fosamil - Acetic Acid/Sodium Acetate Ion Mixtures**

The effect of acetate ion molarity on the one hour kinetic solubility of ceftaroline fosamil was measured at room temperature (25 °C) by adding an excess of ceftaroline fosamil to different USP buffers with pH ranging from 1.2 to 9.0 at acetate ion ionic strengths ranging from 0.1 M to 2.0 M. The results are shown in Tables 6-9.

**TABLE 6: pH Solubility Profile of Ceftaroline Fosamil - Acetic Acid/Sodium Acetate Ion Mixtures (0.1 M)**

| pH | Solubility at 1 hr (mg/mL) |
|---|---|
| 3.0 | 20 |
| 3.8 | 35 |
| 4.6 | 36 |
| 6.0 | 36 |
| 6.7 | 31 |
| 8.2 | 31 |
| 9.4 | 34 |
| USP Buffer Ranges: pH =1-2: HCl/KCl; pH = 3-5: sodium acetate; pH = 6-9: sodium phosphate | |

**TABLE 7: pH Solubility Profile of Ceftaroline Fosamil - Acetic Acid/Sodium Acetate Ion Mixtures (0.5 M)**

| pH | Solubility at 1 hr (mg/mL) |
|---|---|
| 3.1 | 126 |

(continued)

| pH | Solubility at 1 hr (mg/mL) |
|---|---|
| 4.2 | 158 |
| 4.7 | 156 |
| 5.6 | 123 |
| 6.8 | 133 |
| 8.2 | 125 |
| 8.9 | 50 |
| USP Buffer Ranges: pH =1-2: HCl/KCl; pH = 3-5: sodium acetate; pH = 6-9: sodium phosphate | |

**TABLE 8: pH Solubility Profile of Ceftaroline Fosamil - Acetic Acid/Sodium Acetate Ion Mixtures (1.0 M)**

| **pH** | **Solubility at 1 hr (mg/mL)** |
|---|---|
| 2.9 | 213 |
| 3.9 | 237 |
| 4.8 | 235 |
| 5.7 | 212 |
| 6.9 | 207 |
| 7.7 | 205 |
| 8.9 | 102 |
| USP Buffer Ranges: pH =1-2: HCl/KCl; pH = 3-5: sodium acetate; pH = 6-9: sodium phosphate | |

**TABLE 9: pH Solubility Profile of Ceftaroline Fosamil - Acetic Acid/Sodium Acetate Ion Mixtures (2.0 M)**

| pH | Solubility at 1 hr (mg/mL) |
|---|---|
| 3.2 | 152 |
| 3.9 | 273 |
| 4.7 | 271 |
| 5.9 | 244 |
| 6.9 | 225 |
| 7.8 | 212 |
| 9.0 | 159 |
| USP Buffer Ranges: pH =1-2: HCl/KCl; pH = 3-5: sodium acetate; pH = 6-9: sodium phosphate | |

As can be seen from Tables 6-9, the solubility of ceftaroline fosamil increases significantly as the acetate ion ionic strength increases (e.g., the solubility is greater than 200 mg/mL at acetate concentrations of 1.0 M and higher).

**Example 3: Solubility of Ceftaroline Fosamil - Citric Acid/ Sodium Citrate Ion Mixtures**

The effect of citrate ion molarity on the one hour kinetic solubility of ceftaroline fosamil was measured at room temperature (25 °C) by adding an excess of ceftaroline fosamil to different USP buffers at citrate ion ionic strengths ranging from 0.05 M to 1.0 M. The results are shown in Table 10.

**TABLE 10: Solubility Profile of Ceftaroline Fosamil - Citric Acid/Sodium Citrate Ion Mixtures**

| Molarity | Solubility at 1 hr (mg/mL) |
|---|---|
| 0.05 | 121 |
| 0.1 | 159 |
| 0.5 | 240 |
| 1.0 | 245 |

As can be seen from Table 10, the solubility of ceftaroline fosamil increases significantly as the citrate ion ionic strength increases (e.g., the solubility is greater than 200 mg/mL at citrate concentrations of 0.5 M and higher).

**Example 4: Solubility of Ceftaroline Fosamil - DL Arginine Mixtures**

The effect of DL arginine molarity on the one hour kinetic solubility of ceftaroline fosamil was measured at room temperature (25 °C) by adding an excess of ceftaroline fosamil to different USP buffers at DL arginine ionic strengths ranging from 0.05 M to 2.0 M. The results are shown in Table 11.

**TABLE 11: Solubility Profile of Ceftaroline Fosamil - DL Arginine Mixtures**

| Molarity | Solubility at 1 hr (mg/mL) |
|---|---|
| 0.05 | 13 |
| 0.1 | 19 |
| 0.5 | 142 |
| 1.0 | 233 |
| 2.0 | 226 |

As can be seen from Table 11, the solubility of ceftaroline fosamil increases significantly as the DL arginine ionic strength increases (e.g., the solubility is greater than 200 mg/mL at DL arginine concentrations of 1.0 M and higher).

**Example 4: Solubility of Ceftaroline Fosamil - L Arginine Mixtures**

The effect of L-arginine molarity on the one hour kinetic solubility of ceftaroline fosamil was measured at room temperature (25 °C) by adding an excess of ceftaroline fosamil to different USP buffers at L-arginine ionic strengths ranging from 0.05 M to 0.5 M. The results are shown in Table 12.

**TABLE 12: Solubility Profile of Ceftaroline Fosamil - L-Arginine Mixtures**

| Molarity | Solubility at 1 hr (mg/mL) |
|---|---|
| 0.05 | 39 |
| 0.1 | 82 |
| 0.5 | 226 |

As can be seen from Table 12, the solubility of ceftaroline fosamil increases significantly as the L-arginine ionic strength increases (e.g., the solubility is greater than 200 mg/mL at L-arginine concentrations of 0.5 M and higher).

**Example 5: Solubility of Ceftaroline Fosamil - Histidine Mixtures**

The effect of histidine molarity on the one hour kinetic solubility of ceftaroline fosamil was measured at room temperature (25 °C) by adding an excess of ceftaroline fosamil to different USP buffers at histidine ionic strengths ranging from 0.05 M to 0.1 M. The results are shown in Table 13.

**TABLE 13: Solubility Profile of Ceftaroline Fosamil - Histidine Mixtures**

| Molarity | Solubility at 1 hr (mg/mL) |
|---|---|
| 0.05 | 43 |
| 0.1 | 75 |

As can be seen from Table 13, the solubility of ceftaroline fosamil increases as the histidine ion ionic strength increases. The solubility of ceftaroline fosamil at histidine concentrations above 0.1 M could not be determined due to insolubility of histidine in the mixture.

**Example 6: Stability of Solutions Containing Ceftaroline Fosamil/L-Arginine**

A formulation containing 668 mg ceftaroline fosamil and 400 mg L-arginine was prepared. The aqueous solution stability of this formulation (at a concentration of 338 mg ceftaroline fosamil anhydrous, acetate free corrected basis per mL) was determined under the following conditions: (i) 25 °C and (ii) 2-8 °C. The results of these studies are set forth in Tables 14 and 15, respectively.

**TABLE 14: Stability Of Ceftaroline Fosamil/L-Arginine Solution) at 25 °C**

| Time (hours) | % Active of Initial |
|---|---|
| Initial | 100 |
| 0.5 | 100 |
| 1 | 99 |
| 3 | 97 |
| 6 | 96 |
| 24 | 83 |

As can be seen from Table 14, the ceftaroline fosamil/L-arginine solution is stable at room temperature for more than 6 hours, and is therefore suitable for IM administration.

**TABLE 15: Stability Of Ceftaroline Fosamil/L-Arginine Solution at 2-8 °C**

| Time (hours) | % Active of Initial |
|---|---|
| Initial | 100 |
| 24 | 106 |
| 48 | 102 |
| 168 (7 days) | 96 |
| 336 (14 days) | 71 |

The stability of the ceftaroline fosamil/L-arginine solution in a 250 mL home infusion bag was determined under the following conditions: (i) 25 °C and ambient relative humidity (RH) and (ii) 2-8 °C at ambient RH. The results are set forth in Tables 16 and 17, respectively.

**TABLE 16: Stability Of Ceftaroline Fosamil Solution (= 530 mg Ceftaroline) in 250 mL Home Infusion Bag at 25 °C**

| Time (hours) | % Active of Initial |
|---|---|
| Initial | 107 |
| 3 | 106 |
| 6 | 105 |
| 24 | 101 |

(continued)

| Time (hours) | % Active of Initial |
|---|---|
| 48 | 92 |
| 72 | 85 |

**TABLE 17: Stability Of Ceftaroline Fosamil Solution (= 530 mg Ceftaroline) in 250 mL Home Infusion Bag at 2-8 °C**

| Time (hours) | % Active of Initial |
|---|---|
| Initial | 107 |
| 6 | 106 |
| 24 | 110 |
| 48 | 107 |
| 72 | 105 |

As can be seen from Tables 16 and 17, the ceftaroline fosamil/L-arginine solution is stable at room temperature for several days and is therefore suitable for IV infusion use.

The chemical stability of a ceftaroline fosamil/L-arginine blend in 0.9% sodium chloride IV bags was determined at ceftaroline fosamil concentrations of approximately 5 mg/mL under refrigerated (2-8 °C) conditions for 24 or 48 hours followed by 6 hours at ambient conditions (25 °C and ambient light). The results are shown in Table 18.

**TABLE 18: Chemical Stability**

| | Initial (t=0) | 24 hrs at 2-8 °C then 6 hours at 25 °C | 48 hrs at 2-8 °C then 6 hours at 25 °C |
|---|---|---|---|
| Appearance | Clear, colorless | Clear, very slightly yellow | Clear, slightly yellow |
| pH | 6.12 | 6.13 | 6.16 |
| Content (% of initial) | 100 | 97.26 | 97.87 |

As can be seen from Table 18, the pH of the solution over the course of the study was unchanged. Thus the solution may be used for intravenous administration.

**Example 7: Preparation of Formulations Suitable for IM and IV Administration**

Examples of formulations suitable for IM or IV administration are provided in Tables 19-22.

**TABLE 19:**

| Ingredient | Example 1 (mg) | Example 2 (mg) | Example 3 (mg) |
|---|---|---|---|
| Ceftroline Fosamil* | 668 | 446 | 223 |
| L-Arginine | 400 | 267 | 133 |
| * 668 mg ceftaroline fosamil (USAN) is equivalent to about 530 mg ceftaroline; 446 mg ceftaroline fosamil (USAN) is equivalent to about 353 mg ceftaroline; 223 mg ceftaroline fosamil (USAN) is equivalent to 176 mg ceftaroline. | | | |

**TABLE 20:**

| Ingredient | Example 1 (mg) | Example 2 (mg) | Example 3 (mg) |
|---|---|---|---|
| Ceftroline Fosamil* | 668 | 446 | 223 |

(continued)

| Ingredient | Example 1 (mg) | Example 2 (mg) | Example 3 (mg) |
|---|---|---|---|
| DL-Arginine | 400 | 267 | 133 |
| * 668 mg ceftaroline fosamil (USAN) is equivalent to about 530 mg ceftaroline; 446 mg ceftaroline fosamil (USAN) is equivalent to about 353 mg ceftaroline; 223 mg ceftaroline fosamil (USAN) is equivalent to 176 mg ceftaroline. | | | |

**TABLE 21:**

| Ingredient | Example 1 (mg) | Example 2 (mg) | Example 3 (mg) |
|---|---|---|---|
| Ceftroline Fosamil* | 668 | 446 | 223 |
| Citric acid/ Na Citrate | 440 | 293 | 147 |
| * 668 mg ceftaroline fosamil (USAN) is equivalent to about 530 mg ceftaroline; 446 mg ceftaroline fosamil (USAN) is equivalent to about 353 mg ceftaroline; 223 mg ceftaroline fosamil (USAN) is equivalent to 176 mg ceftaroline. | | | |

**TABLE 22:**

| Ingredient | Example 1 (mg) | Example 2 (mg) | Example 3 (mg) |
|---|---|---|---|
| Ceftroline Fosamil* | 668 | 446 | 223 |
| Acetic Acid/Na Acetate | 120 | 82 | 41 |
| * 668 mg ceftaroline fosamil (USAN) is equivalent to about 530 mg ceftaroline; 446 mg ceftaroline fosamil (USAN) is equivalent to about 353 mg ceftaroline; 223 mg ceftaroline fosamil (USAN) is equivalent to 176 mg ceftaroline. | | | |

Multiple strength solutions suitable for IM or IV administration may be prepared from the ceftaroline fosamil/L-arginine blend of Example 1 of Table 19 (i.e., 668 mg ceftaroline fosamil (USAN) (equivalent of 530 mg ceftaroline) and 400 mg L-arginine) according to the following procedures:

<u>223 mg Ceftaroline Fosamil (USAN) for IM Administration:</u>

Add about 2 mL of sterile water for injection to the blend. The resulting solution (2.6 mL) has a concentration equivalent of about 228 mg ceftaroline anhydrous, acetate free corrected basis /mL. Administer about 0.88 mL (equivalent to about 177 mg of ceftaroline).

<u>446 mg Ceftaroline Fosamil (USAN) for IM Administration</u>

Add about 2 mL of sterile water for injection to the blend. The resulting solution has a concentration of about 228 mg ceftaroline anhydrous, acetate free corrected basis /mL. Administer about 1.75 mL (equivalent to about 353 mg of ceftaroline).

<u>668 mg Ceftaroline Fosamil (USAN) for IM Administration</u>

Add about 3 mL of sterile water for injection to the blend. The resulting solution (about 3.6 ml) has a concentration of about 165 mg ceftaroline anhydrous, acetate free corrected basis /mL (equivalent to about 530 mg of ceftaroline).

<u>668 mg Ceftaroline Fosamil (USAN) For IM Administration</u>

Add about 2 mL of sterile water for injection to the blend. The resulting solution has a concentration of about 228 mg ceftaroline anhydrous, acetate free corrected basis /mL. Administer about 2.6 mL (equivalent to about 530 mg of ceftaroline)..

1114 mg Ceftaroline Fosamil (USAN) for IM Administration

Add about 2 mL of sterile water for injection to two vials containing the blend. The resulting solution has a concentration of about 228 mg ceftaroline anhydrous, acetate free corrected basis /mL. Administer about 4.4 mL solution (equivalent to about 883 mg of ceftaroline).

For administration of higher doses, e.g. 2005 mg ceftaroline fasomil (USAN), injections can be administered at two intramuscular sites of a patient.

223 mg Ceftaroline Fosamil (USAN) in 250 mL Infusion for IV Administration

Add about 20 mL of sterile water for injection to the blend. Transfer about 6.67 mL to an IV infusion bag, e.g. 250 mL sterile 0.9 % saline or 5 % dextrose (equivalent to about 177 mg of ceftaroline).

446 mg Ceftaroline Fosamil (USAN) in 250 mL Infusion for IV Administration

Add about 20 mL of sterile water for injection to the blend. Transfer about 13.3 mL to an IV infusion bag, e.g. 250 mL sterile 0.9 % saline or 5 % dextrose (equivalent to about 353 mg of ceftaroline).

668 mg Ceftaroline Fosamil (USAN) in 250 mL infusion for IV Administration

Add about 20 mL of sterile water for injection to the blend. Transfer the contents (20 mL) to an IV infusion bag, e.g. 250 mL sterile 0.9 % saline or 5 % dextrose (equivalent to about 530 mg of ceftaroline).

1337 mg Ceftaroline Fosamil in 250 mL Infusion for IV Administration

Add about 20 mL of sterile water for injection to the blend using two vials. Transfer the contents to an IV infusion bag, e.g. 250 mL sterile 0.9 % saline or 5 % dextrose (equivalent to about 1060 mg of ceftaroline).

2005 mg Ceftaroline Fosamil in 250 mL Infusion for IV Administration

Add about 20 mL of sterile water for injection to the blends using three vials. Transfer the contents to an IV infusion bag, e.g. 250 mL sterile 0.9 % saline or 5 % dextrose (equivalent to about 1589 mg of ceftaroline).

**EXAMPLE 8: A Randomized, Two Part, Single- and Multiple Dose Study of Ceftaraoline Fosamil Parenterally Administered by Intramuscular Injection and Intravenous Injection in Healthy Human Subjects**

This was a two-part randomized, single and multiple-dose study to determine pharmacokinetics of ceftaroline fosamil and its the active moiety, ceftaroline, administered parenterally (both intramuscular injection and intravenous injection).

Part A of the study was a single-dose open-label study. Part B of the study was a multiple-dose study.

**Part A**

Twenty four subjects (six subjects per treatment group) were randomized to one of four treatment groups (A-D): The following doses were administered using vials containing 668 mg ceftaroline fosamil (USAN, molecular formula $C_{22}H_{21}N_8O_8PS_4 \,.\, C_2H_4O_2 \,.\, H_2O$, molecular weight 762.75).

| | |
|---|---|
| Group A: | Day 1 - Single IM injection of 400 mg ceftaroline fosamil (anhydrous, acetate free basis, 228 mg /mL solution) (≡ 353 mg ceftaroline) |
| Group B: | Day 1 - Single IM injection of 600mg ceftaroline fosamil (anhydrous, acetate free basis, 165 mg/mL solution) (≡ 530 mg ceftaroline) |
| Group C: | Day 1 - Single IM injection of 600 mg ceftaroline fosamil (anhydrous, acetate free basis, 228 mg /mL solution) (≡ 530 mg ceftaroline) |
| | Day 8 - Single IV infusion (over 60 mins) of 600 mg ceftaroline fosamil (anhydrous, acetate free basis) (≡ 530 mg ceftaroline) |

Subjects in Treatment Group C received the IV infusion on Day 8

(continued)

Group D: Day 1 - Single IM injection of 1000 mg ceftaroline fosamil (anhydrous, acetate free basis, 228 mg /mL solution) (≡ 883 mg ceftaroline)

**Part B**

Eighteen subjects were randomized to one of two treatment groups The IM injection was be administered to the alternating side of the gluteus in each subject.

Group E: Days 1-4 - IM injection of 600 mg ceftaroline fosamil (anhydrous, acetate free basis 228 mg /mL solution) (≡ 530 mg ceftaroline) every 12 hours
Day 5 - single IM injection of 600 mg ceftaroline fosamil (anhydrous, acetate free basis 228 mg /mL solution) (≡ 530 mg ceftaroline) 12 hours post Day 4 last dose

Control Group F: Days 1-4 - IM injection of cefepime hydrochloride 1000 mg every 12 hours
Day 5 - a single IM injection of cefepime hydrochloride 1000 mg 12 hours post Day 4 last dose was used as a control group

The duration of the study for Treatment Groups E and F was 11 days (Days -1 through 10). The volume of fluid administered for each intramuscular injection is summarized in Table 23. Ceftaroline dosing solutions for IM injection were prepared using sterile water for injection.

**TABLE 23: Volumes for IM Injection**

| Treatment Group | IM Ceftaroline Fosamil (USAN) Dose administered* | Equivalent Ceftaroline Fosamil (INN) Dose | Concentration (in terms of ceftaroline fosamil anhydrous, acetate free basis INN, mg/mL) | Approximate Volume Injected (mL) |
|---|---|---|---|---|
| A | 446 mg | 400 mg | 228 | 1.8 |
| B | 668 mg | 600 mg | 165 | 3.6 |
| C | 668 mg | 600 mg | 228 | 2.6 |
| D | 1114 mg | 1000 mg | 228 | 4.4 |
| E | 668 mg | 600 mg | 228 | 2.6 |
| * 668 mg ceftaroline fosamil (USAN) is equivalent to about 530 mg ceftaroline; 446 mg ceftaroline fosamil (USAN) is equivalent to about 353 mg ceftaroline and 1114 mg ceftaroline fosamil (USAN) is equivalent to about 883 mg ceftaroline. | | | | |

The pharmacokinetic parameters for ceftaroline fosamil and ceftaroline were determined using a standard assay. Blood for pharmacokinetic parameter analysis was collected from all subjects as follows: The first blood sample was collected at about 5 minutes (about 0.06 hours) after completion of the administration. Measurement was considered essentially zero prior to this point.

**Part A:** 5, 15, and 30 minutes and 1, 2, 4, 6, 8, 12, 18, 24, 36 and 48 hours after injection. In addition, blood were be collected from subjects receiving IV infusion immediately prior to drug injection on day 8 and at 20, 40, 60 (immediately before the end of the study-drug infusion), 65 and 75 minutes, and 1.5, 2, 3, 4, 6, 8, 12, 24, 36 and 48 hours after the start of the drug infusion on Day 8.

**Part B:** Drug injection on Days 1 and 5, at 5, 15, and 30 minutes and 1, 2, 4, 6, 8 and hours after the first injection on Day 1, immediately prior to (within 15 minutes) of injection of morning dose on Day 4, at 12 hours post Day 4 morning dose (before evening dose) and at 5, 15 and 30 minutes and 1, 2, 4, 6, 8, 12, 24, 36 and 48 hours after the last (morning) injection on Day 5.

Pharmacokinetic parameters for ceftaroline fosamil obtained from Part A of this study are presented in Table 24.

**TABLE 24: Mean Pharmacokinetic Parameters for Ceftaroline Fosamil INN (Prodrug)**

| PK Parameter for Ceftaroline Fosamil (INN) | Treatment C 600 mg (INN) IV (Day 8) | Treatment C 600 mg (INN) IM (Day 1) (228 mg/mL) | Treatment A 400 mg (INN) IM ((228 mg/mL) | Treatment B 600 mg (INN) IM (165 mg/mL) | Treatment D 1000 mg (INN) IM (228 mg/mL) |
|---|---|---|---|---|---|
| $C_{max}$ (ng/mL) | 3549 ± 465 | 1492 ± 275 | 1113 ± 418 | 2722 ± 557 | 2239 ± 657 |
| $T_{max}$ (h) | 0.67 (0.33-0.98) | 0.50 (0.08-1.0) | 0.29 (0.08-1.0) | 0.75 (0.25-1.0) | 0.38 (0.08-1.0) |
| $AUC_{0-t}$ (hr.ng/mL) | 2734 ± 340 | 3060 ± 475 | 1950 ± 401 | 4846 ± 975 | 5791 ± 2795 |
| $AUC_{0-\infty}$ (hr.ng/mL) | 2744 ± 344 | 3242 ± 486 | 2144 ± 421 | 5118 ± 985 | 6229 ± 2829 |
| $T_{1/2}$ (h) | 0.07 ± 0.03 | 1.57 ± 0.41 | 1.21 ± 0.49 | 1.06 ± 0.33 | 1.87 ± 0.90 |

The $AUC_{0-\infty}$ and $C_{max}$ values increase or decrease proportionally with the dosage of ceftaroline fosamil. Thus, one skilled in the art with the benefit of this disclosure may readily determine pharmacokinetic parameters for any specific dosage of ceftaroline fosamil (or other prodrugs of ceftaroline) used in a particular dosage form of the invention. The pro-drug in the blood is converted rapidly to the active moiety ceftaroline.

Pharmacokinetic parameters for ceftaroline obtained from Part A of this study are presented in Table 25.

**TABLE 25: Mean Pharmacokinetic Parameters for Ceftaroline (molecular formula $C_{22}H_{22}N_8O_5S_4$, Active Moiety)**

| PK Parameter for Ceftaroline (Active Moiety) | Treatment C 600 mg (INN) IV (Day 8) | Treatment C 600 mg (INN) IM (Day 1) (228 mg/mL) | Treatment A 400 mg (INN) IM (228 mg/mL) | Treatment B 600 mg (INN) IM (165 mg/mL) | Treatment D 1000 mg (INN) IM (228 mg/mL) |
|---|---|---|---|---|---|
| $C_{max}$ (ng/mL) | 19685 ± 2264 | 8510 ± 1691 | 6971 ± 1616 | 14651 ± 3299 | 15997 ± 3739 |
| $T_{max}$ (h) | 1.0 (1.0-1.0) | 2.0 (1.2-2.0) | 1.5 (1.0-2.0) | 2.0 (1.0-2.0) | 2.0 (1.0-2.0) |
| $AUC_{0-t}$ (hr.ng/mL) | 44587 ± 5026 | 47466 ± 4006 | 35285 ± 6155 | 73439 ± 12080 | 109893 ± 31345 |
| $AUC_{0-\infty}$ (hr.ng/mL) | 44987 ± 5041 | 48108 ± 3846 | 35611 ± 6131 | 73826 ± 12031 | 110265 ± 31283 |
| $T_{1/2}$ (h) | 2.13 ± 0.31 | 2.55 ± 0.49 | 2.36 ± 0.22 | 2.27 ± 0.16 | 2.68 ± 0.31 |
| Bioavailability (%) | NA | 107 ± 7.1 | NA | NA | NA |

The doses were administered using vials containing 668 mg ceftaroline fasomil (USAN, molecular formula $C_{22}H_{21}N_8O_8PS_4 . C_2H_4O_2 . H_2O$, molecular weight 762.75). As can be seen from Table 25, the systemic exposure ($AUC_{0-\infty}$) of ceftaroline (active moiety) following dosing with equivalent of 600 mg ceftaroline fosamil (INN) (≡ 530 mg ceftaroline) by IM injection at a concentration of 228 mg/mL defined per Table 23 (Day 1) is approximately equivalent to the systemic exposure following an IV infusion of equivalent of 600 mg ceftaroline fosamil (INN) (≡ 530 mg ceftaroline) (Day 8), resulting in an absolute bioavailability of approximately 100%. The $C_{max}$ value of ceftaroline for IM injection in Treatment Group C is approximately 57% lower than the $C_{max}$ value following IV infusion. The $T_{max}$ following IM injection is about 1 to 2 hours, while the $T_{max}$ following IV infusion occurred around the time of the end of the infusion (~ 1 hour).

Also, as can be seen from Table 25, plasma concentrations following IM injection using vials containing 668 mg ceftaroline fosamil (USAN, molecular formula $C_{22}H_{21}N_8O_8PS_4 . C_2H_4O_2 . H_2O$, molecular weight 762.75, which is equivalent to 600 mg ceftaroline fosamil (INN) (= 530 mg ceftaroline) at a concentration of 165 mg/mL (defined per Table 23) were greater than following IM injection of 600 mg ceftaroline fosamil (INN) (≡ 530mg ceftaroline) at a concentration of 228 mg/mL (defined per Table 23), resulting in $C_{max}$ and AUC values that were approximately 72% and 56%. The IM solution containing the equivalent of 600 mg ceftaroline fosamil (INN) at a concentration of 165 mg/mL (defined per Table 23) is over 50% more bioavailable that an IV solution of the same strength. This is unexpected.

Linear calculated pharmacokinetic parameters for ceftaroline following single dose IM injection of ceftaroline

fosamil using vials containing 668 mg ceftaroline fosamil (USAN) at 228 mg/mL, defined per Table 23 are shown in Table 26.

**TABLE 26: Linear Calculated Pharmacokinetic Parameters for Ceftaroline**

| Dose Ceftaroline Fo(USAN) | Equivalent Ceftaroline Dose | Mean $C_{max}$ (ng/mL) | Mean $AUC_{0-\infty}$ (ng/mL * h) | Mean $T_{max}$ (hr) |
|---|---|---|---|---|
| 223 mg | 177 mg | 3486 | 17806 | 1.5 |
| 446 mg | 353 mg | 6971 | 35611 | 1.5 |
| 557 mg | 442 mg | 7899 | 43163 | 2 |
| 668 mg | 530 mg | 8510 | 48108 | 2 |
| 891 mg | 706 mg | 12570 | 81803 | 2 |
| 1114 mg | 883 mg | 15977 | 110265 | 2 |
| 1337 mg | 1060 mg | 18798 | 133323 | 2 |
| 2005 mg | 1589 mg | 28140 | 210603 | 2 |
| * Ceftaroline values for 177, 442, 706, 1060 and 1589 mg are calculated based on linear assumption | | | | |

Linear calculated pharmacokinetic parameters for ceftaroline fosamil (INN) following single dose IM injection of ceftaroline fosamil USAN (at concentration of ceftaroline fosamil (anhydrous, acetate free basis of 228 mg /mL, defined per Table 23) are shown in Table 27.

**TABLE 27: Linear Calculated Pharmacokinetic Parameters for Ceftaroline Fosamil (INN)**

| Ceftaroline Fasomil (USAN) | Equivalent Ceftaroline Fasomil (INN) Dose | Mean $C_{max}$ (ng/mL) | Mean $AUC_{0-\infty}$ (ng/mL * h) | Mean $T_{max}$ (hr) |
|---|---|---|---|---|
| 223 | 200 | 484 | 1162 | 0.3 |
| 446 | 400 | 1113 | 2144 | 0.29 |
| 557 | 500 | 1210 | 2905 | 0.5 |
| 668 | 600 | 1492 | 3242 | 0.5 |
| 891 | 800 | 1936 | 4648 | 0.5 |
| 1114 | 1000 | 2238 | 6229 | 0.5 |
| 1337 | 1200 | 2904 | 6972 | 0.5 |
| 2005 | 1800 | 4356 | 10458 | 0.5 |
| Dose is in terms of amount of ceftaroline, for example, 668 mg of ceftaroline fosamil (USAN) is equivalent to 530 mg ceftaroline. Values for ceftaroline fosamil (USAN) 223, 557, 891, 1337 and 2005 mg are calculated based on linear assumptions | | | | |

Pharmacokinetic parameters for ceftaroline obtained from Part B of this study are presented in Table 28.

**TABLE 28: Mean Pharmacokinetic Parameters for Ceftaroline (Molecular Formula: $C_{22}H_{22}N_8O_5S_4$)**

| PK Parameter Ceftaroline (Active Moiety) | Treatment E 600 mg (INN) IM (Day 1) 228 mg/mL | Treatment E 600 mg (INN) IM (Day 5) q12h 228 mg/mL |
|---|---|---|
| $C_{max}$ (ng/mL) | 11557 ± 3416 | 12960 1361 |
| $T_{max}$ (h) | 2.0 (11.0-2.02) | 2.0 (1.0-2.02) |
| $AUC_{0-t}$ (hr.ng/mL) | 55289 ± 11076 | 65407 ± 11807 |

(continued)

| PK Parameter Ceftaroline (Active Moiety) | Treatment E 600 mg (INN) IM (Day 1) 228 mg/mL | Treatment E 600 mg (INN) IM (Day 5) q12h 228 mg/mL |
|---|---|---|
| $T_{1/2}$ (h) | 2.54 ± 0.63 | 2.51 ± 0.45 |
| Dose is in terms of amount of ceftaroline, for example, 668 mg of ceftaroline fosamil (USAN) is equivalent to 530 mg ceftaroline | | |

Pharmacokinetic parameters for ceftaroline fosamil obtained from Part B of this study are presented in Table 29.

**TABLE 29: Mean Pharmacokinetic parameters for Ceftaroline Fosamil (INN)**

| PK Parameter Ceftaroline Fosamil (INN) | Treatment E 600 mg (INN) IM (Day 1) 228 mg/mL | Treatment E 600 mg (INN) IM (Day 5) q12h 228 mg/mL |
|---|---|---|
| $C_{max}$ (ng/mL) | 1575 ± 507 | 2223 ± 497 |
| $T_{max}$ (h) | 0.50 (0.08-1.0) | 0.50 (0.08-1.0) |
| $AUC_{0-t}$ (hr.ng/mL) | 3096 ± 850 | 4067 ± 782 |
| $T_{1/2}$ (h) | 1.17 ± 0.52 | 1.11 ± 0.35 |
| Dose is in terms of amount of ceftaroline, for example, 668 mg of ceftaroline fosamil (USAN) is equivalent to 530 mg ceftaroline | | |

While the invention has been depicted and described by reference to exemplary embodiments of the invention, such a reference does not imply a limitation on the invention, and no such limitation is to be inferred. The invention is capable of considerable modification, alteration, and equivalents in form and function, as will occur to those ordinarily skilled in the pertinent arts having the benefit of this disclosure. The depicted and described embodiments of the invention are exemplary only, and are not exhaustive of the scope of the invention. Consequently, the invention is intended to be limited only by the spirit and scope of the appended claims, giving full cognizance to equivalence in all respects.

The entire disclosures of all patents, patent applications and publications, cited herein, are hereby incorporated by reference.

## Claims

**1.** A dosage form comprising ceftaroline, or a pharmaceutically acceptable salt and/or solvate thereof and/or prodrug thereof, and a solubilizing agent, wherein the molarity of the solubilizing agent in an aqueous solution of the dosage form is greater than about 0.1 M.

**2.** The dosage form of claim 1, wherein the molarity of the solubilizing agent is greater than about 0.5 M.

**3.** The dosage form of claim 1, wherein the molarity of the solubilizing agent is greater than about 1.0 M.

**4.** The dosage form of claim 1, comprising ceftaroline fosamil, wherein the ceftaroline fosamil is ceftaroline fosamil-monoacetate monohydrate (USAN) or ceftaroline fosamil-anhydrous acetate free (INN).

**5.** The dosage form of claim 4, wherein the ceftaroline fosamil has an aqueous solubility of greater than about 40 mg/mL.

**6.** The dosage form of claim 4, wherein the ceftaroline fosamil has an aqueous solubility of greater than about 100 mg/mL.

**7.** The dosage form of claim 4, wherein the ceftaroline fosamil has an aqueous solubility of greater than about 200 mg/mL.

**8.** The dosage form of claim 1, wherein the solubilizing agent is selected from carboxylic acids and amino acids.

**9.** The dosage form of claim 1, wherein the solubilizing agent is selected from the group consisting of formic acid, acetic acid, propionic acid, butyric acid, valeric acid, caproic acid, enanthic acid, caprylic acid, pelargonic acid, capric acid, lauric acid, stearic acid, acrylic acid, docosahexaenoci acid, eicosapentaenoic acid, pyruvic acid, benzoic acid, salicylic acid, aldaric acid, oxalic acid, malonic acid, malic acid, succinic acid, glutaric acid, adipic acid, citric acid, lactic acid, alanine, arginine, aspargine, aspartic acid, cysteine, , glutamine, glycine, histidine, isoleucine, leucine, lysine, methionine, phenylalanine, praline, serine, threonine, tryptophan, tyrosine, valine, and salts thereof and combinations thereof.

**10.** The dosage form of claim 9, wherein the solubilizing agent is selected from L-arginine, DL-arginine, citric acid and salts thereof, acetic acid and salts thereof, histadine, and combinations thereof.

**11.** The dosage form of claim 10, wherein the solubilizing agent is L-arginine.

**12.** The dosage form of claim 10, wherein the solubilizing agent is citric acid/sodium citrate.

**13.** The dosage form of claim 10, wherein the solubilizing agent is acetic acid/sodium acetate.

**14.** A dosage form comprising from about 223 mg to about 2005 mg of ceftaroline fosamil, wherein a single dose parenteral administration of the dosage form provides an *in vivo* plasma profile for ceftaroline comprising a mean $AUC_{0-\infty}$ of more than about 10,650 ng.hr/mL.

**15.** The dosage form of claim 14, wherein a IM single dose administration of the dosage form provides an *in vivo* plasma profile for ceftaroline comprising
a mean $C_{max}$ of less than about 39,500 ng/mL, and
a mean $AUC_{0-\infty}$ of more than about 10,650 ng.hr/mL.

**16.** The dosage form of claim 14, wherein a single dose IM administration of the dosage form provides an *in vivo* plasma profile for ceftaroline comprising
a mean $C_{max}$ of less than about 39,500 ng/mL, and
a mean $AUC_{0-\infty}$ of more than about 10,650 ng.hr/mL, and
a mean $T_{max}$ of about 1 or more hours.

**17.** The dosage form of claim 14, comprising 223 mg ceftaroline fosamil, wherein a single dose parenteral administration of the dosage form provides an *in vivo* plasma profile for ceftaroline comprising a mean $AUC_{0-\infty}$ of more than about 10,650 ng.hr/mL.

**18.** The dosage form of claim 17, wherein a single dose IM administration of the dosage form provides an *in vivo* plasma profile for ceftaroline comprising
a mean $C_{max}$ of less than about 4,900 ng/mL, and
a mean $AUC_{0-\infty}$ of more than about 10,650 ng.hr/mL.

**19.** The dosage form of claim 17, wherein a single dose IM administration of the dosage form provides an *in vivo* plasma profile for ceftaroline comprising
a mean $C_{max}$ of less than about 4,900 ng/mL,
a mean $AUC_{0-\infty}$ of more than about 10,650 ng.hr/mL, and
a mean $T_{max}$ of about 1 or more hours.

**20.** The dosage form of claim 14, comprising 446 mg ceftaroline fosamil, wherein a single dose parenteral administration of the dosage form provides an *in vivo* plasma profile for ceftaroline comprising a mean $AUC_{0-\infty}$ of more than about 21,350 ng.hr/mL.

**21.** The dosage form of claim 20, wherein a single dose IM administration of the dosage form provides an *in vivo* plasma profile for ceftaroline comprising
a mean $C_{max}$ of less than about 9,800 ng/mL, and
a mean $AUC_{0-\infty}$ of more than about 21,350 ng.hr/mL.

**22.** The dosage form of claim 20, wherein a single dose IM administration of the dosage form provides an *in vivo* plasma profile for ceftaroline comprising

a mean $C_{max}$ of less than about 9,800 ng/mL,
a mean $AUC_{0-\infty}$ of more than about 21,350 ng.hr/mL, and
a mean $T_{max}$ of about 1 or more hours.

**23.** The dosage form of claim 14, comprising 557 mg ceftaroline fosamil, wherein a single dose parenteral administration of the dosage form provides an *in vivo* plasma profile for ceftaroline comprising a mean $AUC_{0-\infty}$ of more than about 25,800 ng.hr/mL.

**24.** The dosage form of claim 23, wherein a single dose IM administration of the dosage form provides an *in vivo* plasma profile for ceftaroline comprising
a mean $C_{max}$ of less than about 11,100 ng/mL, and
a mean $AUC_{0-\infty}$ of more than about 25,800 ng.hr/mL.

**25.** The dosage form of claim 23, wherein a single dose IM administration of the dosage form provides an *in vivo* plasma profile for ceftaroline comprising
a mean $C_{max}$ of less than about 11,100 ng/mL,
a mean $AUC_{0-\infty}$ of more than about 25,800 ng.hr/mL, and
a mean $T_{max}$ of about 1 or more hours.

**26.** The dosage form of claim 14, comprising 668 mg ceftaroline fosamil, wherein a single dose parenteral administration of the dosage form provides an *in vivo* plasma profile for ceftaroline comprising a mean $AUC_{0-\infty}$ of more than about 28,800 ng.hr/mL.

**27.** The dosage form of claim 26, wherein a single dose IM administration of the dosage form provides an *in vivo* plasma profile for ceftaroline comprising
a mean $C_{max}$ of less than about 12,000 ng/mL, and
a mean $AUC_{0-\infty}$ of more than about 28,800 ng.hr/mL.

**28.** The dosage form of claim 26, wherein a single dose IM administration of the dosage form provides an *in vivo* plasma profile for ceftaroline comprising
a mean $C_{max}$ of less than about 12,000 ng/mL,
a mean $AUC_{0-\infty}$ of more than about 28,800 ng.hr/mL, and
a mean $T_{max}$ of about 1 or more hours.

**29.** The dosage form of claim 14, comprising 891 mg ceftaroline fosamil, wherein a single dose parenteral administration of the dosage form provides an *in vivo* plasma profile ceftaroline comprising a mean $AUC_{0-\infty}$ of more than about 49,000 ng.hr/mL.

**30.** The dosage form of claim 29, wherein a single dose IM administration of the dosage form provides an *in vivo* plasma profile for ceftaroline comprising
a mean $C_{max}$ of less than about 17,750 ng/mL, and
a mean $AUC_{0-\infty}$ of more than about 49,000 ng.hr/mL.

**31.** The dosage form of claim 29, wherein a single dose IM administration of the dosage form provides an *in vivo* plasma profile for ceftaroline comprising
a mean $C_{max}$ of less than about 17,750 ng/mL,
a mean $AUC_{0-\infty}$ of more than about 49,000 ng.hr/mL, and
a mean $T_{max}$ of about 1 or more hours.

**32.** The dosage form of claim 14, comprising 1114 mg ceftaroline fosamil, wherein a single dose parenteral administration of the dosage form provides an *in vivo* plasma profile for ceftaroline comprising a mean $AUC_{0-\infty}$ of more than about 66,000 ng.hr/mL.

**33.** The dosage form of claim 32, wherein a single dose IM administration of the dosage form provides an *in vivo* plasma profile for ceftaroline comprising
a mean $C_{max}$ of less than about 22,500 ng/mL, and
a mean $AUC_{0-\infty}$ of more than about 66,000 ng.hr/mL.

**34.** The dosage form of claim 32, wherein a single dose IM administration of the dosage form provides an *in vivo* plasma profile for ceftaroline comprising
a mean $C_{max}$ of less than about 22,500 ng/mL,
a mean $AUC_{0-\infty}$ of more than about 66,000 ng.hr/mL, and
a mean $T_{max}$ of about 1 or more hours.

**35.** The dosage form of claim 14, comprising 1337 mg ceftaroline fosamil, wherein a single dose parenteral administration of the dosage form provides an *in vivo* plasma profile for ceftaroline comprising a mean $AUC_{0-\infty}$ of more than about 79,500 ng.hr/mL.

**36.** The dosage form of claim 35, wherein a single dose IM administration of the dosage form provides an *in vivo* plasma profile for ceftaroline comprising
a mean $C_{max}$ of less than about 26,500 ng/mL, and
a mean $AUC_{0-\infty}$ of more than about 79,500 ng.hr/mL.

**37.** The dosage form of claim 35, wherein a single dose IM administration of the dosage form provides an *in vivo* plasma profile for ceftaroline comprising
a mean $C_{max}$ of less than about 26,500 ng/mL,
a mean $AUC_{0-\infty}$ of more than about 79,500 ng.hr/mL, and
a mean $T_{max}$ of about 1 or more hours.

**38.** The dosage form of claim 14, comprising 2005 mg ceftaroline fosamil, wherein a single dose parenteral administration of the dosage form provides an *in vivo* plasma profile for ceftaroline comprising a mean $AUC_{0-\infty}$ of more than about 126,000 ng.hr/mL.

**39.** The dosage form of claim 38, wherein a single dose IM administration of the dosage form provides an *in vivo* plasma profile for ceftaroline comprising
a mean $C_{max}$ of less than about 39,500 ng/mL, and
a mean $AUC_{0-\infty}$ of more than about 126,000 ng.hr/mL.

**40.** The dosage form of claim 38, wherein a single dose IM administration of the dosage form provides an *in vivo* plasma profile for ceftaroline comprising
a mean $C_{max}$ of less than about 39,500 ng/mL,
a mean $AUC_{0-\infty}$ of more than about 126,000 ng.hr/mL, and
a mean $T_{max}$ of about 1 or more hours.

**41.** A dosage form comprising about 668 mg ceftaroline fosamil and about 400 mg L-arginine, wherein the ceftaroline fosamil has an aqueous solubility of greater than about 100 mg/mL.

**42.** A dosage form comprising about 668 mg ceftaroline fosamil and about 348 mg L-arginine, wherein the ceftaroline fosamil has an aqueous solubility of greater than about 100 mg/mL.

**43.** A dosage form comprising about 668 mg ceftaroline fosamil and about 174 mg L-arginine, wherein the ceftaroline fosamil has an aqueous solubility of greater than about 100 mg/mL.

**44.** A dosage form comprising about 446 mg ceftaroline fosamil and about 267 mg L-arginine, wherein the ceftaroline fosamil has an aqueous solubility of greater than about 100 mg/mL.

**45.** A dosage form comprising about 446 mg ceftaroline fosamil and about 230 mg L-arginine, wherein the ceftaroline fosamil has an aqueous solubility of greater than about 100 mg/mL.

**46.** A dosage form comprising about 446 mg ceftaroline fosamil and about 116 mg L-arginine, wherein the ceftaroline fosamil has an aqueous solubility of greater than about 100 mg/mL.

**47.** The dosage form of any one of claims 1-46 in the form of a powder

**48.** The dosage form of any one of claims 1-46 in the form of a solution or suspension in a solvent.

**49.** The dosage form of claim 45, wherein the solvent is selected from water, physiological saline, an about 5-10% glucose or dextrose solution, and combinations thereof.

**50.** A method of treating a bacterial infection, comprising administering to a patient in need thereof, an effective amount of a dosage form according to claim 48.

**51.** The method according to claim 14, wherein the dosage form is administered intramuscularly.

**52.** The method according to claim 14, wherein the dosage form is administered intravenously.

Europäisches Patentamt
European Patent Office
Office européen des brevets

# EUROPEAN SEARCH REPORT

Application Number
EP 15 16 6135

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 310 502 A1 (TAKEDA CHEMICAL INDUSTRIES LTD [JP]) 14 May 2003 (2003-05-14) * paragraphs [0019], [0020]; examples 11-14 * | 1-52 | INV. A61K31/546 A61P31/04 |
| X | EP 1 618 894 A1 (TAKEDA PHARMACEUTICAL [JP]) 25 January 2006 (2006-01-25) * examples 8-11 * | 1-52 | |
| X | ISHIKAWA TOMOYASU ET AL: "TAK-599, a novel N-phosphono type prodrug of anti-MRSA cephalosporin T-91825: synthesis, physicochemical and pharmacological properties.", 29 May 2003 (2003-05-29), BIOORGANIC & MEDICINAL CHEMISTRY 29 MAY 2003 LNKD-PUBMED:12735989, VOL. 11, NR. 11, PAGE(S) 2427 - 2437, XP002599200, ISSN: 0968-0896 | 14-40 | |
| Y | * abstract * * page 2431 - page 2432 * | 1-52 | TECHNICAL FIELDS SEARCHED (IPC) |
| X | PRESCOTT L M ET AL: "46th Interscience Conference on Antimicrobial Agents and Chemotherapy", P & T PHARMACY AND THERAPEUTICS JOURNAL, MEDIMEDIA, US, vol. 31, no. 11, 1 November 2006 (2006-11-01), pages 679-682, XP009138237, ISSN: 1052-1372 | 14-40 | A61K A61P |
| Y | * page 679 * | 1-52 | |
| | -/-- | | |

1

The present search report has been drawn up for all claims

| Place of search | Date of completion of the search | Examiner |
|---|---|---|
| Munich | 3 November 2015 | Zimmer, Barbara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 16 6135

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BARBHAIYA R H ET AL: "Safety, tolerance, and pharmacokinetics of cefepime administered intramuscularly to healthy subjects.", JOURNAL OF CLINICAL PHARMACOLOGY OCT 1990 LNKD- PUBMED:2229450, vol. 30, no. 10, October 1990 (1990-10), pages 900-910, XP009138236, ISSN: 0091-2700<br>* page 901, right-hand column, paragraph 3 *<br>* page 910, left-hand column, last paragraph *<br>----- | 1-52 | |
| Y | EP 0 695 548 A1 (MEIJI SEIKA KAISHA [JP]) 7 February 1996 (1996-02-07)<br>* page 1, lines 5-8 *<br>* page 2, lines 47-53; examples *<br>----- | 1-52 | |
| Y | US 2003/039956 A1 (CHOI SEUNG-HO [US] ET AL) 27 February 2003 (2003-02-27)<br>* paragraph [0097] *<br>----- | 1-52 | TECHNICAL FIELDS SEARCHED (IPC) |

1

The present search report has been drawn up for all claims

| Place of search | Date of completion of the search | Examiner |
|---|---|---|
| Munich | 3 November 2015 | Zimmer, Barbara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 16 6135

This annex lists the patent family members relating to the patent documents cited in the above-mentioned European search report. The members are as contained in the European Patent Office EDP file on
The European Patent Office is in no way liable for these particulars which are merely given for the purpose of information.

03-11-2015

| Patent document cited in search report | Publication date | Patent family member(s) | Publication date |
|---|---|---|---|
| EP 1310502 A1 | 14-05-2003 | AT 431827 T | 15-06-2009 |
| | | AU 7775301 A | 25-02-2002 |
| | | CA 2418614 A1 | 04-02-2003 |
| | | CN 1462275 A | 17-12-2003 |
| | | CY 1109313 T1 | 02-07-2014 |
| | | DK 1310502 T3 | 07-09-2009 |
| | | EP 1310502 A1 | 14-05-2003 |
| | | EP 2020416 A1 | 04-02-2009 |
| | | ES 2323759 T3 | 24-07-2009 |
| | | KR 20030022891 A | 17-03-2003 |
| | | PT 1310502 E | 31-07-2009 |
| | | US 2004023943 A1 | 05-02-2004 |
| | | US 2005176697 A1 | 11-08-2005 |
| | | WO 0214333 A1 | 21-02-2002 |
| EP 1618894 A1 | 25-01-2006 | EP 1618894 A1 | 25-01-2006 |
| | | KR 20060013378 A | 09-02-2006 |
| | | TW I344847 B | 11-07-2011 |
| | | WO 2004096279 A1 | 11-11-2004 |
| EP 0695548 A1 | 07-02-1996 | BR 9503522 A | 04-06-1996 |
| | | CA 2155170 A1 | 04-02-1996 |
| | | CN 1119531 A | 03-04-1996 |
| | | EP 0695548 A1 | 07-02-1996 |
| | | JP H0840907 A | 13-02-1996 |
| | | US 5595986 A | 21-01-1997 |
| US 2003039956 A1 | 27-02-2003 | US 2003039956 A1 | 27-02-2003 |
| | | US 2009264340 A1 | 22-10-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 97419407 P
- US 6417175 B
- US 6906055 B


### Non-patent literature cited in the description


- *WHO Drug Information,* 2007, vol. 21 (2
- Developing Antimicrobial Drugs - General Considerations for Clinical Trials. Draft Guidance for Industry. U.S. Department of Health and Human Services, July 1998